(19) Europäisches Patentamt
European Patent Office
Office européen des brevets

(11) EP 2 945 710 B1

(12) **EUROPEAN PATENT SPECIFICATION**

(45) Date of publication and mention of the grant of the patent:
**08.11.2017 Bulletin 2017/45**

(21) Application number: **14700917.9**

(22) Date of filing: **20.01.2014**

(51) Int Cl.:
***A61Q 17/04*** *(2006.01)* ***A61K 8/06*** *(2006.01)*
***A61K 8/41*** *(2006.01)* ***A61K 8/81*** *(2006.01)*

(86) International application number:
**PCT/EP2014/051019**

(87) International publication number:
**WO 2014/111571 (24.07.2014 Gazette 2014/30)**

# (54) COSMETIC OR DERMATOLOGICAL EMULSION COMPRISING A MEROCYANINE AND AN EMULSIFYING SYSTEM CONTAINING AN AMPHIPHILIC POLYMER COMPRISING AT LEAST ONE 2-ACRYLAMIDOMETHYLPROPANESULFONIC ACID UNIT

KOSMETISCHE ODER DERMATOLOGISCHE EMULSION MIT EINEM MEROCYANIN UND EINEM EMULGIERUNGSSYSTEM MIT EINEM AMPHIPHILEN POLYMER MIT MINDESTENS EINER 2-ACRYLAMIDOMETHYLPROPANSULFONSÄUREEINHEIT

ÉMULSION COSMÉTIQUE OU DERMATOLOGIQUE COMPRENANT UNE MÉROCYANINE ET UN SYSTÈME ÉMULSIFIANT CONTENANT UN POLYMÈRE AMPHIPHILE COMPRENANT AU MOINS UNE UNITÉ D'ACIDE 2-ACRYLAMIDOMETHYLPROPANESULFONIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.01.2013 FR 1350489**

(43) Date of publication of application:
**25.11.2015 Bulletin 2015/48**

(73) Proprietor: **L'Oréal**
**75008 Paris (FR)**

(72) Inventors:
- **ROUDOT, Angelina**
**F-94270 Le Kremlin Bicêtre (FR)**
- **SUIDA-BATISTA, Alexandra**
**F-91800 Brunoy (FR)**
- **CANDAU, Didier**
**F-91570 Bièvres (FR)**
- **LALLORET, Florence**
**F-75014 Paris (FR)**

(74) Representative: **Le Blainvaux Bellegarde, Françoise**
**L'Oréal**
**Service DIPI**
**9 Rue Pierre Dreyfus**
**92110 Clichy (FR)**

(56) References cited:
**EP-A1- 1 069 142**
**EP-A1- 1 728 501**
**WO-A1-2004/006878**
**WO-A1-2011/113718**
**WO-A2-2009/027258**
**WO-A2-2013/011094**
**DE-A1-102005 059 738**
**DE-A1-102007 061 969**
**FR-A1- 2 835 432**
**FR-A1- 2 939 651**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 945 710 B1

## Description

The present invention relates to a cosmetic or dermatological composition in emulsion form, comprising, in a physiologically acceptable support:

a) at least one aqueous phase, and
b) at least one oily phase, and
c) at least one merocyanine compound of formula (1) which will be defined in greater detail herein below, and
d) at least one emulsifying system containing at least one amphiphilic polymer comprising at least one 2-acrylamidomethylpropanesulfonic acid unit.

Another subject of the present invention consists of a non-therapeutic cosmetic process for caring for and/or making up a keratin material, comprising the application, to the surface of the said keratin material, of at least one composition according to the invention as defined above.

The invention also relates to a non-therapeutic cosmetic process for limiting the darkening of the skin and/or improving the colour and/or uniformity of the complexion, comprising the application, to the surface of the keratin material, of at least one composition as defined previously.

The invention also relates to a non-therapeutic cosmetic process for preventing and/or treating the signs of ageing of a keratin material, comprising the application, to the surface of the keratin material, of at least one composition as defined previously.

It is known that radiation with wavelengths of between 280 nm and 400 nm permits tanning of the human epidermis and that radiation with wavelengths of between 280 and 320 nm, known as UVB rays, harms the development of a natural tan. Exposure is also liable to bring about a detrimental change in the biomechanical properties of the epidermis, which is reflected by the appearance of wrinkles, leading to premature ageing of the skin.

It is also known that UVA rays with wavelengths of between 320 and 400 nm penetrate more deeply into the skin than UVB rays. UVA rays cause immediate and persistent browning of the skin. Daily exposure to UVA rays, even of short duration, under normal conditions can result in damage to the collagen fibres and the elastin, which is reflected by a modification in the microrelief of the skin, the appearance of wrinkles and uneven pigmentation (liver spots, lack of uniformity of the complexion).

Protection against UVA and UVB rays is thus necessary. An efficient photoprotective product should protect against both UVA and UVB rays.

Many photoprotective compositions have been proposed to date to overcome the effects induced by UVA and/or UVB rays. They generally contain organic and/or mineral UV-screening agents, which function according to their own chemical nature and according to their own properties by absorption, reflection or scattering of the UV rays. They generally comprise mixtures of liposoluble organic screening agents and/or water-soluble UV-screening agents in combination with metal oxide pigments, such as titanium dioxide or zinc oxide.

Many cosmetic compositions for limiting the darkening of the skin and improving the colour and uniformity of the complexion have been proposed to date. It is well known in the field of antisun products that such compositions may be obtained by using UV-screening agents, and in particular UVB-screening agents. Certain compositions may also contain UVA-screening agents. This screening system should cover UVB protection for the purpose of limiting and controlling the neosynthesis of melanin, which promotes the overall pigmentation, but should also cover UVA protection so as to limit and control the oxidation of the already-existing melanin leading to darkening of the skin colour.

However, no composition contains a particular combination of UV-screening agents that would be especially suited to photoprotecting the skin and particularly to improving the quality of the skin as regards both the colour and its mechanical elasticity properties.

Advantageously, this improvement is particularly visible on already-pigmented skin so as not to increase the melanin pigmentary load or the structure of the melanin already present in the skin.

In point of fact, the majority of the organic UV-screening agents consist of aromatic compounds which absorb in the wavelength range between 280 and 370 nm. In addition to their power for screening out sunlight, the desired photoprotective compounds should also have good cosmetic properties, good solubility in the usual solvents and in particular in fatty substances such as oils, and also good chemical stability and good photostability alone or in combination with other UV-screening agents. They should also be colourless or at least have a colour that is cosmetically acceptable to the consumer.

One of the main drawbacks known to date of these compositions is that these screening systems are insufficiently effective against UV rays and in particular against long UVA rays with wavelengths beyond 370 nm, for the purpose of controlling photo-induced pigmentation and its evolution by means of a system for screening out UV over the entire UV spectrum.

Among all the compounds that have been recommended for this purpose an advantageous family of UV-screening agents has been proposed, which consists of carbon-bearing merocyanine derivatives, which is described in patent US 4 195 999, patent application WO 2004/006 878, patent applications WO2008/090066, WO2011/113718, WO2009/027258, WO2013/010590, WO2013/011094, WO20130/11480 and the documents IP COM JOURNAL N°000179675D published on February 23, 2009, IP COM JOURNAL N°000182396D published on April 29, IP COM JOURNAL N° 000189542D published on November 12, 2009, IP COM Journal N°IPCOM000011179D published on 03/04/2004. Some of these compounds may show the following drawbacks :

- relatively unsatisfactory solubility in the usual solvents and in particular in fatty substances such as oils which may require a laborious formulation process and/or may result in cosmetic drawbacks such as a greasy effect on application ;
- an unsatisfactory chemical stability and/or unsatisfactory photostability
- produce a color liable to discourage the consumer from using a cosmetic or dermatological composition containing them.

Antisun compositions are quite often in the form of an emulsion of oil-in-water type (i.e. a cosmetically acceptable support consisting of a continuous aqueous dispersing phase and of a discontinuous oily dispersed phase) or of the water-in-oil type (i.e. a cosmetically acceptable support consisting of a continuous oily dispersing phase and of a discontinuous aqueous dispersed phase) which contains, in varying concentrations, one or more conventional lipophilic and/or hydrophilic organic screening agents which are capable of selectively absorbing harmful UV rays, these screening agents (and the amounts thereof) being selected as a function of the desired sun protection factor.

EP-A1-1728501 discloses storage stable compositions comprising polypeptide-bound UV-A filters as well as other UV filters.

DE-A1-102005059738 discloses sunscreen compositions with high UV-A balance comprising merocyanines. The Applicant has found in the course of its research that some of the merocyanines could lose their efficacy in the presence of certain particular emulsifiers in antisun compositions. The Applicant has especially observed that some of these merocyanines degrade chemically in the presence of certain emulsifying systems as those constituted of at least one alkali metal salt of a phosphoric acid ester of a fatty alcohol.

There is thus still a need to select other families of emulsifiers which guarantee a good chemical stability of the merocyanines without the drawbacks as previously defined.

The Applicant has discovered, surprisingly, that the use of one emulsifying system containing at least one amphiphilic polymer comprising at least one 2-acrylamidomethylpropanesulfonic acid unit and one merocyanine compound of formula (1) make it possible to achieve this objective.

Furthermore, the merocyanine compounds of formula (1) herein below, present surprinsingly the advantage to be significantly less colored than the merocyanine compounds as disclosed in the application WO2008/090066 as the compound MC11 also called MC03 in the application WO2009/027258.

Those discoveries form the basis of the present invention.

Thus, in accordance with one of the objects of the present invention, a cosmetic or dermatological composition in emulsion form is now proposed, comprising, in a physiologically acceptable support:

a) at least one aqueous phase, and
b) at least one oily phase, and
c) at least one merocyanine compound of formula (1) which will be defined in greater detail herein below, and
d) at least one emulsifying system containing at least one amphiphilic polymer comprising at least one 2-acrylamidomethylpropanesulfonic acid unit.

Another subject of the present invention consists of a non-therapeutic cosmetic process for caring for and/or making up a keratin material, comprising the application, to the surface of the said keratin material, of at least one composition according to the invention as defined above.

The invention also relates to a non-therapeutic cosmetic process for limiting the darkening of the skin and/or improving the colour and/or uniformity of the complexion, comprising the application, to the surface of the keratin material, of at least one composition as defined previously.

The invention also relates to a non-therapeutic cosmetic process for preventing and/or treating the signs of ageing of a keratin material, comprising the application, to the surface of the keratin material, of at least one composition as defined previously.

Other characteristics, aspects and advantages of the invention will emerge on reading the detailed description that follows.

The expression "human keratin materials" means the skin (body, face, area around the eyes), hair, eyelashes, eyebrows, body hair, nails, lips or mucous membranes.

The term "physiologically acceptable" means compatible with the skin and/or its integuments, having a pleasant colour, odour and feel and not causing any unacceptable discomfort (stinging, tautness or redness) liable to discourage the consumer from using this composition.

The term "between X and Y" means the range of values also including the limits X and Y.

According to the invention, the term "preventing" or "prevention" means reducing the risk of occurrence or slowing down the occurrence of a given phenomenon, namely, according to the present invention, the signs of ageing of a keratin material.

The term "emulsion" means any macroscopically homogeneous, kinetically stable composition comprising at least two mutually immiscible phases; one being a dispersing continuous phase and the other being dispersed in the said continuous phase in the form of droplets. The two phases are kinetically stabilized by at least one emulsifying system generally comprising at least one emulsifying surfactant.

Emulsions are distinguished as being of the oil-in-water type, known as "direct" emulsions, consisting of an aqueous dispersing continuous phase and of an oily dispersed discontinuous phase, and emulsions of the water-in-oil type, known as inverse emulsions, consisting of an oily dispersing continuous phase and of an aqueous dispersed discontinuous phase. There are also multiple emulsions, for instance water-in-oil-in-water or oil-in-water-in-oil emulsions.

The term "emulsifying system" refers to any compound or mixture of compounds that is capable of increasing the kinetic stability of an emulsion. These compounds are generally amphiphilic and are surfactants characterized by their more or less hydrophilic or more or less lipophilic nature which will determine their ability to stabilize direct emulsions or inverse emulsions. They are especially classified by their HLB according to the calculation method of W.C. Griffin in the document "Classification of Surface Active Agents by HLB, Journal of the Society of Cosmetic Chemists 1 (1949) 311" and in the document "Calculation of HLB of Non Ionic Surfactants, Journal of the Society of Cosmetic Chemists 5 (1954) 249". The calculation of the HLB according to this calculation method is performed according to the equation:

$$HLB = 20 \times Mh/M$$

where Mh is the molar mass of the hydrophilic part of the surfactant and M is the total molecular mass of the molecule.

**<u>MEROCYANINES</u>**

According to the present invention, the merocyanine compounds in accordance with the invention correspond to formula (1) below, and also the E/E- or E/Z-geometrical isomer forms thereof:

(1) 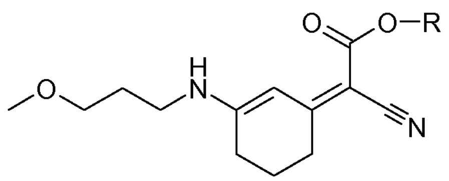

in which:

R is a $C_1$-$C_{22}$ alkyl group, a $C_2$-$C_{22}$ alkenyl group, a $C_2$-$C_{22}$ alkynyl group, a $C_3$-$C_{22}$ cycloalkyl group or a $C_3$-$C_{22}$ cycloalkenyl group, the said groups possibly being interrupted with one or more O.

The merocyanine compounds of the invention may be in their E/E- or E/Z-geometrical isomer forms.

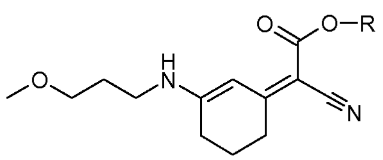

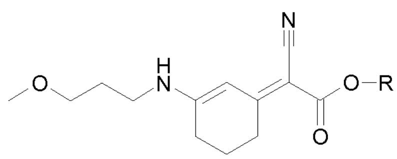

The preferential compounds of formula (1) are those in which:

R is a $C_1$-$C_{22}$ alkyl, which may be interrupted with one or more O.

Among the compounds of formula (1), use will be made more particularly of those chosen from the following compounds, and also the E/E- or E/Z- geometrical isomer forms thereof:

| | | | |
|---|---|---|---|
| 1 | 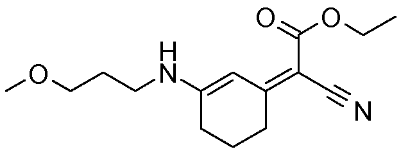<br>ethyl (2Z)-cyano{3-[(3-methoxypropyl)amino]cyclohex-2-en-1-ylidene} ethanoate | 4 | 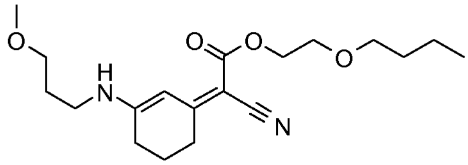<br>2-butoxyethyl (2Z)-cyano{3-[(3-methoxypropyl)amino]cyclohex-2-en-1-ylidene} ethanoate |
| 2 | 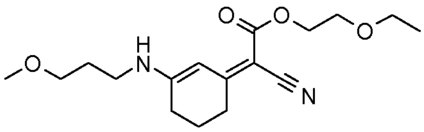<br>2-ethoxyethyl (2Z)-cyano{3-[(3-methoxypropyl)amino]cyclohex-2-en-1-ylidene} ethanoate | 5 | 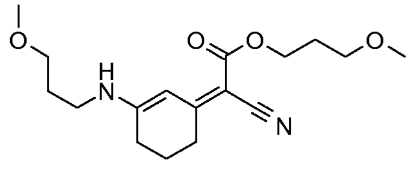<br>3-methoxypropyl (2Z)-cyano{3-[(3-methoxypropyl)amino]cyclohex-2-en-1-ylidene} ethanoate |
| 3 | 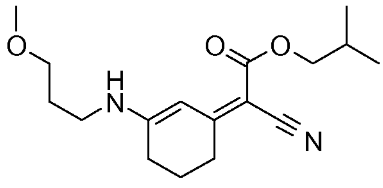<br>2-methylpropyl (2Z)-cyano{3-[(3-methoxypropyl)amino]cyclohex-2-en-1-ylidene} ethanoate | 6 | 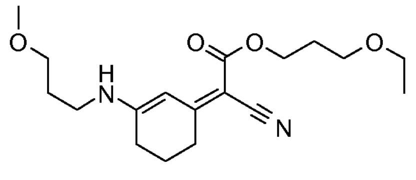<br>3-ethoxypropyl (2Z)-cyano{3-[(3-methoxypropyl)amino]cyclohex-2-en-1-ylidene} ethanoate |

According to a more particularly preferred mode of the invention, use will be made of the compound 2-ethoxyethyl (2Z)-cyano{3-[(3-methoxypropyl)amino]cyclohex-2-en-1-ylidene}ethanoate (2) in its E/Z geometrical configuration having the following structure:

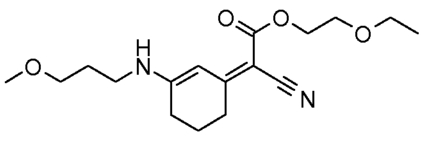

and/or in its E/E geometrical configuration having the following structure:

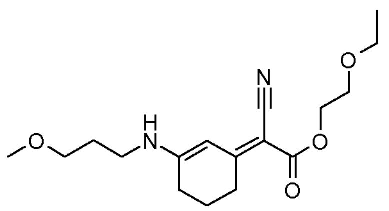

The merocyanines of formula (1) according to the invention are preferably present in the compositions according to the invention in a concentration ranging from 0.1 % to 10% by weight and preferentially from 0.2% to 5% by weight relative to the total weight of the composition.

The compounds of formula (1) may be prepared according to the protocols described in Pat. Appl. WO 2007/071 582, in IP.com Journal (2009), 9(5A), 29-30 IPCOM000182396D under the title "Process for producing 3-amino-2-cyclohexan-1-ylidene compounds" and in US-A-4 749 643 on column 13, line 66 - column 14, line 57 and the references cited in this regard.

## EMULSIFYING SYSTEM

### a) Amphiphilic polymer comprising at least one 2-acrylamidomethylpropanesulfonic acid unit

For the purposes of the invention, the term "amphiphilic polymer" is intended to denote a polymer having amphiphilic properties, i.e. having at least one hydrophilic part and at least one hydrophobic part. Hydrophilic groups and hydrophobic groups are well known to those skilled in the art.

For the purposes of the present invention, the term "polymer" is intended to denote a compound comprising at least two repeating units and in particular at least five repeating units.

The amphiphilic polymers under consideration according to the invention are amphiphilic polymers comprising at least one 2-acrylamidomethylpropanesulfonic acid (AMPS®) unit.

The amphiphilic polymers comprising at least one 2-acrylamidomethylpropanesulfonic acid (AMPS®) unit that may be used in the present invention, which are also known more simply as "amphiphilic AMPS® polymers" hereinbelow, comprise both a hydrophilic part and a hydrophobic part comprising at least one fatty chain.

The fatty chain present in the said amphiphilic AMPS® polymers according to the invention may preferably comprise from 7 to 30 carbon atoms and more preferentially from 7 to 22 carbon atoms.

The amphiphilic AMPS® polymers according to the invention are especially chosen from amphiphilic polymers of at least one 2-acrylamidomethylpropanesulfonic acid (AMPS®) monomer and of at least one ethylenically unsaturated hydrophobic comonomer comprising at least one hydrophobic part containing from 7 to 30 carbon atoms and in particular from 7 to 22 carbon atoms or even from 12 to 22 carbon atoms.

The amphiphilic AMPS® polymers according to the invention generally have a weight-average molecular weight ranging from 50 000 to 10 000 000 g/mol, in particular from 100 000 to 8 000 000 g/mol and even more particularly from 100 000 to 7 000 000 g/mol.

They may be crosslinked or non-crosslinked.

When the amphiphilic AMPS® polymers according to the invention are crosslinked, the crosslinking agents may be chosen from the polyolefinically unsaturated compounds commonly used for the crosslinking of polymers obtained by free-radical polymerization.

Examples of crosslinking agents that may be mentioned include divinylbenzene, diallyl ether, dipropylene glycol diallyl ether, polyglycol diallyl ethers, triethylene glycol divinyl ether, hydroquinone diallyl ether, ethylene glycol di(meth)acrylate, tetraethylene glycol di(meth)acrylate, trimethylolpropane triacrylate, methylenebis(acrylamide), methylenebis(methacrylamide), triallylamine, triallyl cyanurate, diallyl maleate, tetraallylethylenediamine, tetraallyloxyethane, trimethylolpropane diallyl ether, allyl (meth)acrylate, allylic ethers of alcohols of the sugar series, or other allylic or vinyl ethers of polyfunctional alcohols, and also allylic esters of phosphoric and/or vinylphosphonic acid derivatives, or mixtures of these compounds.

The crosslinking agents may be chosen especially from methylenebis(acrylamide), allyl methacrylate and trimethylolpropane triacrylate (TMPTA).

The degree of crosslinking may range, for example, from 0.01 mol% to 10 mol% and preferably from 0.2 mol% to 2 mol% relative to the polymer.

The amphiphilic AMPS® polymers according to the invention may be chosen especially from random amphiphilic AMPS® polymers modified by reaction with a $C_6$-$C_{22}$ n-monoalkylamine or di-n-alkylamine, such as those described in patent application WO 00/31154.

An amphiphilic polymer that is suitable for use in the invention may comprise at least one ethylenically unsaturated hydrophilic comonomer chosen, for example, from acrylic acid, methacrylic acid or substituted alkyl derivatives thereof or esters thereof obtained with monoalkylene or polyalkylene glycols, acrylamide, methacrylamide, vinylpyrrolidone, vinylformamide, maleic anhydride, itaconic acid or maleic acid, or mixtures thereof.

An amphiphilic polymer according to the invention may comprise at least one ethylenically unsaturated hydrophobic comonomer.

An amphiphilic polymer that is suitable for use in the invention may comprise at least one hydrophobic part chosen from saturated or unsaturated, linear alkyl radicals, for instance n-octyl, n-decyl, n-hexadecyl, n-dodecyl and oleyl, branched alkyl radicals, for instance isostearyl, or cyclic alkyl radicals, for instance cyclododecane or adamantane.

An amphiphilic AMPS® polymer may also contain at least one ethylenically unsaturated hydrophobic comonomer comprising, for example:

- a fluoro or $C_7$-$C_{18}$ fluoroalkyl radical (for example the group of formula $-(CH_2)_2-(CF_2)_9-CF_3$),
- a cholesteryl radical or a cholesterol-based radical (for example cholesteryl hexanoate),

- a polycyclic aromatic group, for instance naphthalene or pyrene,
- a silicone, alkylsilicone or alkylfluorosilicone radical.

These copolymers are especially described in document EP-A-0 750 899, patent US-A-5 089 578 and in the following publications by Yotaro Morishima:

- Self-assembling amphiphilic polyelectrolytes and their nanostructures - Chinese Journal of Polymer Science Vol. 18, No. 40, (2000), 323-336;
- Micelle formation of random copolymers of sodium 2-(acrylamido)-2-methylpropanesulfonate and a nonionic surfactant macromonomer in water as studied by fluorescence and dynamic light scattering - Macromolecules 2000, Vol. 33, No. 10 - 3694-3704;
- Solution properties of micelle networks formed by nonionic moieties covalently bound to a polyelectrolyte: salt effects on rheological behavior - Langmuir, 2000, Vol. 16, No. 12, 5324-5332;
- Stimuli responsive amphiphilic copolymers of sodium 2-(acrylamido)-2-methylpropanesulfonate and associative macromonomers - Polym. Preprint, Div. Polym. Chem. (1999), 40(2), 220-221.

They are also described in documents EP 1 069 142, WO 02/44224, WO 02/44225, WO 02/44227, WO 02/44229, WO 02/44230, WO 02/44231, WO 02/44267, WO 02/44268, WO 02/44269, WO 02/44270, WO 02/44271, WO 02/43677, WO 02/43686, WO 02/43687, WO 02/43688 and WO 02/43689, in the name of Clariant.

An ethylenically unsaturated hydrophobic comonomer of the invention may preferably be chosen from the acrylates or acrylamides of formula (I) below:

(I)

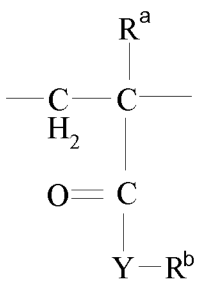

in which:

- $R^a$ denotes a hydrogen atom or a linear or branched $C_1$-$C_6$ alkyl radical, preferably methyl;
- Y denotes O or NH;
- $R^b$ denotes a hydrophobic radical comprising a fatty chain containing from 7 to 30 carbon atoms, preferably from 7 to 22 and more particularly from 12 to 22 carbon atoms.

The hydrophobic radical $R^b$ is chosen from saturated or unsaturated linear $C_7$-$C_{22}$ alkyl radicals (for example n-octyl, n-decyl, n-hexadecyl, n-dodecyl or oleyl), branched alkyl radicals (for example isostearic) or cyclic alkyl radicals (for example cyclododecane or adamantane); $C_7$-$C_{18}$ alkylperfluoro radicals (for example the group of formula $-(CH_2)_2(CF_2)_9-CF_3$); the cholesteryl radical or a cholesterol ester, for instance cholesteryl hexanoate; aromatic polycyclic groups, for instance naphthalene or pyrene.

Among these radicals, linear and branched alkyl radicals are more particularly preferred.

According to a preferred embodiment of the invention, the hydrophobic radical $R^b$ may also comprise at least one alkylene oxide unit and preferably a polyoxyalkylene chain.

The polyoxyalkylene chain may preferentially consist of ethylene oxide units and/or propylene oxide units and even more particularly consists solely of ethylene oxide units.

The number of moles of oxyalkylene units may generally range from 1 to 30 mol, more preferentially from 1 to 25 mol and even more preferentially from 3 to 20 mol.

Among these polymers, mention may be made of:

- crosslinked or noncrosslinked, neutralized or non-neutralized copolymers comprising from 15% to 60% by weight of AMPS® units and from 40% to 85% by weight of ($C_8$-$C_{16}$)alkyl(meth)acrylamide units or of ($C_8$-$C_{16}$)alkyl (meth)acrylate units relative to the polymer, such as those described in patent application EP-A-0 750 899;
- terpolymers comprising from 10 mol% to 90 mol% of acrylamide units, from 0.1 mol% to 10 mol% of AMPS® units and from 5 mol% to 80 mol% of n-($C_6$-$C_{18}$)alkylacrylamide units relative to the polymer, such as those described in

patent US-A-5 089 578;

- non-crosslinked copolymers of partially or completely neutralized AMPS® and of n-dodecyl methacrylate, n-hexadecyl methacrylate or n-octadecyl methacrylate, such as those described in the Morishima articles mentioned above;
- crosslinked or non-crosslinked copolymers of partially or completely neutralized AMPS® and of n-dodecylmethacrylamide, such as those described in the Morishima articles mentioned above.

Amphiphilic AMPS® polymers that may also be mentioned include copolymers of totally neutralized AMPS® and of n-dodecyl, n-hexadecyl and/or n-octadecyl methacrylate, and also non-crosslinked and crosslinked copolymers of AMPS® and of n-dodecylmethacrylamide.

Mention will be made more particularly of crosslinked or non-crosslinked amphiphilic AMPS® copolymers consisting of:

2-acrylamido-2-methylpropanesulfonic acid (AMPS®) units of formula (II) below:

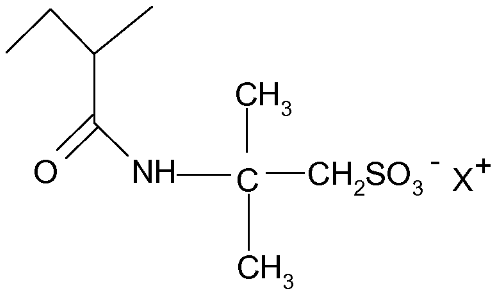

(II)

in which X is a proton, an alkali metal cation, an alkaline-earth metal cation or an ammonium ion;
and units of formula (III) below:

(III)

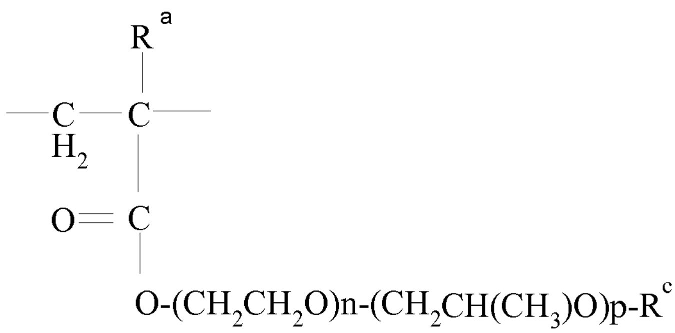

in which n and p, independently of one another, denote a number of moles and range from 0 to 30, preferentially from 1 to 25 and more preferentially from 3 to 20, with the proviso that n + p is less than or equal to 30, preferably less than 25 and better still less than 20; $R^a$ denotes a hydrogen atom or a linear or branched $C_1$-$C_6$ alkyl radical, preferably methyl, and $R^c$ denotes a linear or branched alkyl containing from 7 to 22 carbon atoms and preferably from 12 to 22 carbon atoms.

In formula (II), the cation X more particularly denotes sodium or ammonium.

Among the monomers of formula (III), mention may be made of:

- esters of (meth)acrylic acid and of a $C_{10}$-$C_{18}$ fatty alcohol polyoxyethylenated with 8 mol of ethylene oxide, for instance the product Genapol C-080® sold by the company Clariant,
- esters of (meth)acrylic acid and of a $C_{11}$ fatty oxoalcohol polyoxyethylenated with 8 mol of ethylene oxide, for instance the product Genapol UD-080® sold by the company Clariant,
- esters of (meth)acrylic acid and of a $C_{12}$-$C_{14}$ fatty alcohol polyoxyethylenated with 7 mol of ethylene oxide, for instance the product Genapol LA-070® sold by the company Clariant,
- esters of (meth)acrylic acid and of a $C_{12}$-$C_{14}$ fatty alcohol polyoxyethylenated with 11 mol of ethylene oxide, for instance the product Genapol LA-110® sold by the company Clariant,
- esters of (meth)acrylic acid and of a $C_{16}$-$C_{18}$ fatty alcohol polyoxyethylenated with 8 mol of ethylene oxide, for

instance the product Genapol T-080® sold by the company Clariant,

- esters of (meth)acrylic acid and of a $C_{16}$-$C_{18}$ fatty alcohol polyoxyethylenated with 15 mol of ethylene oxide, for instance the product Genapol T-150® sold by the company Clariant,
- esters of (meth)acrylic acid and of a $C_{16}$-$C_{18}$ fatty alcohol polyoxyethylenated with 11 mol of ethylene oxide, for instance the product Genapol T-110® sold by the company Clariant,
- esters of (meth)acrylic acid and of a $C_{16}$-$C_{18}$ fatty alcohol polyoxyethylenated with 20 mol of ethylene oxide, for instance the product Genapol T-200® sold by the company Clariant,
- esters of (meth)acrylic acid and of a $C_{16}$-$C_{18}$ fatty alcohol polyoxyethylenated with 25 mol of ethylene oxide, for instance the product Genapol T-250® sold by the company Clariant,
- esters of (meth)acrylic acid and of a $C_{18}$-$C_{22}$ fatty alcohol polyoxyethylenated with 25 mol of ethylene oxide and/or of a $C_{16}$-$C_{18}$ fatty isoalcohol polyoxyethylenated with 25 mol of ethylene oxide.

The products that will be chosen more particularly are:

- the non-crosslinked products for which p = 0, n = 7 or 25, $R^a$ denotes a methyl and $R^c$ represents a mixture of $C_{12}$-$C_{14}$ or $C_{16}$-$C_{18}$ alkyl,
- the crosslinked products for which p = 0, n = 8 or 25, $R^a$ denotes a methyl and $R^c$ represents a mixture of $C_{16}$-$C_{18}$ alkyl.

These polymers are described and synthesized in patent application EP 1 069 142.

These particular amphiphilic AMPS® polymers may be obtained according to the standard processes of free-radical polymerization in the presence of one or more initiators, for instance azobisisobutyronitrile (AIBN), azobisdimethylvaleronitrile, 2,2-azobis(2-amidinopropane) hydrochloride (ABAH), organic peroxides such as dilauryl peroxide, benzoyl peroxide, tert-butyl hydroperoxide, mineral peroxide compounds such as potassium or ammonium persulfate, or $H_2O_2$ optionally in the presence of reducing agents.

These amphiphilic AMPS® polymers may be obtained especially by free-radical polymerization in tert-butanol medium, in which they precipitate. By using precipitation polymerization in tert-butanol, it is possible to obtain a size distribution of the polymer particles that is particularly favourable for its uses.

The reaction may be performed at a temperature of between 0 and 150°C and preferably between 10 and 100°C, either at atmospheric pressure or under reduced pressure.

It may also be performed under inert atmosphere, and preferably under nitrogen.

The amphiphilic AMPS® polymers according to the invention may preferably be partially or totally neutralized with a mineral base such as sodium hydroxide, potassium hydroxide, aqueous ammonia or an organic base such as monoethanolamine, diethanolamine, triethanolamine, an aminomethylpropanediol, N-methylglucamine, basic amino acids, for instance arginine and lysine, and mixtures of these compounds. They may especially be totally or almost totally neutralized, i.e. at least 80% neutralized.

The molar percentage concentration of the units of formula (II) and of the units of formula (III) in the amphiphilic AMPS® polymers according to the invention may vary as a function of the desired cosmetic application, for example the nature of the emulsion (oil-in-water or water-in-oil emulsion) and the desired rheological properties of the formulation. It may range, for example, between 0.1 mol% and 99.9 mol%.

Preferably, for the most hydrophobic polymers, the molar proportion of units of formula (I) or (III) ranges from 50.1% to 99.9%, more particularly from 70% to 95% and even more particularly from 80% to 90%.

Preferably, for the sparingly hydrophobic polymers, the molar proportion of units of formula (I) or (III) ranges from 0.1% to 50%, more particularly from 5% to 25% and even more particularly from 10% to 20%.

The distribution of the monomers in the AMPS® polymers according to the invention may be, for example, alternate, block (including multiblock) or random.

As a guide, and without this being limiting, mention may be made especially of:

- a non-crosslinked copolymer of 2-acrylamidomethylpropanesulfonic acid and of a $C_{12}$-$C_{14}$ alkyl methacrylate polyethoxylated with 25 mol of ethylene oxide (INCI name: Ammonium acryloyldimethyltaurate/laureth-7 methacrylate copolymer) (non-crosslinked copolymer obtained from Genapol LA-070 and AMPS®) sold under the name Aristoflex LNC by the company Clariant,
- a crosslinked copolymer of 2-acrylamidomethylpropanesulfonic acid and of stearyl methacrylate polyethoxylated with 25 mol of ethylene oxide (INCI name: Ammonium acryloyldimethyltaurate/steareth-25 methacrylate crosspolymer) (copolymer crosslinked with trimethylolpropane triacrylate obtained from Genapol T-250 and AMPS®) sold under the name Aristoflex HMS by the company Clariant,
- a non-crosslinked copolymer of 2-acrylamidomethylpropanesulfonic acid and of a $C_{16}$/$C_{18}$ alkyl methacrylate polyethoxylated with 8 mol of ethylene oxide (INCI name: Ammonium acryloyldimethyltaurate/steareth-8 methacrylate copolymer) sold under the name Aristoflex SNC by the company Clariant,

- a crosslinked copolymer of 2-acrylamidomethylpropanesulfonic acid and of behenyl methacrylate polyethoxylated with 25 mol of ethylene oxide, crosslinked with trimethylolpropane triacrylate (INCI name: Ammonium acryloyldimethyltaurate/beheneth-25 methacrylate crosspolymer) sold under the name Aristoflex HMB by the company Clariant, and mixtures thereof.

According to a preferred embodiment, the polymer used according to the invention is a copolymer of AMPS and of a $C_{16}$-$C_{18}$ alkyl methacrylate comprising from 6 to 25 mol of ethylene oxide, this copolymer being obtained from AMPS and methacrylic acid or a methacrylic acid salt and from a polyoxyethylenated $C_{16}$-$C_{18}$ alcohol comprising 6 to 25 mol of ethylene oxide.

According to an even more preferred embodiment of the invention, the polymer used according to the invention is a copolymer of AMPS and of a $C_{16}$-$C_{18}$ alkyl methacrylate comprising from 6 to 10 mol of ethylene oxide, for which the mole proportion of $C_{16}$-$C_{18}$ alkyl methacrylate units (units (II)) ranges from 2% to 15% and better still from 5% to 10%. It is more particularly a copolymer of AMPS and of methacrylic acid or a methacrylic acid salt and of oxyethylenated stearyl alcohol (Genapol T-080) comprising a mole proportion of alkyl methacrylate units of 7.35%.

A preferred polymer according to the invention that may be mentioned in particular is the copolymer of AMPS and of the ammonium salt of methacrylic acid and of oxyethylenated stearyl alcohol comprising 8 mol of ethylene oxide (Genapol T-080 methacrylate) and comprising a mole proportion of alkyl methacrylate units of 7.35%, sold under the name Aristoflex SNC by the company Clariant (INCI name: Ammonium acryloyldimethyltaurate/steareth-8 methacrylate copolymer).

The amphiphilic polymers in accordance with the invention are present in the compositions in active material concentrations preferably ranging from 0.01% to 2% by weight, more preferentially from 0.01% to 1% by weight and better still from 0.1 % to 0.5% by weight, relative to the total weight of the composition.

**b) <u>Additional emulsifiers</u>**

The compositions according to the invention may be in the form of an oil-in-water emulsion or a water-in-oil emulsion as a function of the nature of the relative proportion of the hydrophobic and hydrophilic parts and/or of the chemical nature of the amphiphilic polymer thereof comprising at least one 2-acrylamidomethylpropanesulfonic acid unit.

They may contain other emulsifying surfactants that are suited to the type of emulsion.

For the O/W emulsions, examples of emulsifiers that may be mentioned include nonionic emulsifiers such as oxyalkylenated (more particularly polyoxyethylenated) esters of fatty acids and of glycerol; oxyalkylenated esters of fatty acids and of sorbitan; oxyalkylenated (oxyethylenated and/or oxypropylenated) esters of fatty acids, such as the PEG-100 stearate/glyceryl stearate mixture sold, for example, by ICI under the name Arlacel 165; oxyalkylenated (oxyethylenated and/or oxypropylenated) ethers of fatty alcohols; esters of sugars, such as sucrose stearate; or ethers of fatty alcohol and of sugar, in particular alkyl polyglucosides (APGs), such as decyl glucoside and lauryl glucoside, sold, for example, by the company Henkel under the respective names Plantaren 2000 and Plantaren 1200, cetostearyl glucoside, optionally as a mixture with cetostearyl alcohol, sold, for example, under the name Montanov 68 by the company SEPPIC, under the name Tegocare CG90 by the company Goldschmidt and under the name Emulgade KE3302 by the company Henkel, and also arachidyl glucoside, for example in the form of a mixture of arachidyl alcohol, behenyl alcohol and arachidyl glucoside, sold under the name Montanov 202 by the company SEPPIC. According to a particular embodiment of the invention, the mixture of the alkyl polyglucoside as defined above with the corresponding fatty alcohol may be in the form of a self-emulsifying composition, for example as described in document WO-A-92/06778.

According to a particular form of the invention, use will also be made of $C_{11}$-$C_{18}$ fatty acids such as undecanoic acid or lauric acid, such as the products sold under the trade name Acide Laurique 98® by the company Stéarineries Dubois, or Edenor C12-99 MY® by the company Emery Oleochemicals, palmitic acid, such as the products sold under the trade name Palmera A9516® or Palmera A9816® by the company KLK Oleo, or Acide Palmitique 95% - Pastilles by the company Stéarineries Dubois, or the stearic acid sold under the trade name Palmera B1802CG by the company KLK Oleo, Stéarine TP 1200 Pastilles® (DUB 50P®) by the company Stéarineries Dubois, or Kortacid PH 05.02® by the company Pacific Oleochemicals, or mixtures thereof, for instance the mixture of stearic and palmitic acid sold under the trade name Dub Microlub 50® by the company Stéarineries Dubois, and more particularly stearic acid. The fatty acids may be present in the compositions of the invention preferably between 0.1% and 5% and more particularly between 0.5% and 3%.

As emulsifying surfactants that may be used for the preparation of the W/O emulsions, examples that may be mentioned include sorbitan, glycerol or sugar alkyl esters or ethers; silicone surfactants, for instance dimethicone copolyols, such as the mixture of cyclomethicone and of dimethicone copolyol, sold under the name DC 5225 C by the company Dow Corning, and alkyldimethicone copolyols such as laurylmethicone copolyol sold under the name Dow Corning 5200 Formulation Aid by the company Dow Corning; cetyldimethicone copolyol, such as the product sold under the name Abil EM 90R by the company Goldschmidt, and the mixture of cetyldimethicone copolyol, of polyglyceryl

isostearate (4 mol) and of hexyl laurate, sold under the name Abil WE 09 by the company Goldschmidt. One or more coemulsifiers may also be added thereto, which may be chosen advantageously from the group consisting of polyol alkyl esters.

Polyol alkyl esters that may especially be mentioned include polyethylene glycol esters, for instance PEG-30 dipolyhydroxystearate, such as the product sold under the name Arlacel P135 by the company ICI.

Esters of glycerol and/or of sorbitan that may be mentioned include, for example, polyglyceryl isostearate, such as the product sold under the name Isolan GI 34 by the company Goldschmidt, sorbitan isostearate, such as the product sold under the name Arlacel 987 by the company ICI, sorbitan glyceryl isostearate, such as the product sold under the name Arlacel 986 by the company ICI, and mixtures thereof.

According to a particular mode of the invention, the composition will not contain any emulsifying agents other than the amphiphilic polymer comprising at least one 2-acrylamidomethylpropanesulfonic acid unit.

The term "not containing" means "containing less than 0.5%" or even "free of" emulsifying agents other than the polymer of the invention.

## OILY PHASE

The compositions in accordance with the invention comprise at least one oily phase.

For the purposes of the invention, the term "oily phase" means a phase comprising at least one oil and all of the liposoluble and lipophilic ingredients and the fatty substances used for the formulation of the compositions of the invention.

The term "oil" means any fatty substance that is in liquid form at room temperature (20 - 25°C) and at atmospheric pressure (760 mmHg).

An oil that is suitable for use in the invention may be volatile or non-volatile.

An oil that is suitable for use in the invention may be chosen from hydrocarbon-based oils, silicone oils and fluoro oils, and mixtures thereof.

A hydrocarbon-based oil that is suitable for use in the invention may be an animal hydrocarbon-based oil, a plant hydrocarbon-based oil, a mineral hydrocarbon-based oil or a synthetic hydrocarbon-based oil.

An oil that is suitable for use in the invention may be advantageously chosen from mineral hydrocarbon-based oils, plant hydrocarbon-based oils, synthetic hydrocarbon-based oils and silicone oils, and mixtures thereof.

For the purposes of the present invention, the term "silicone oil" means an oil comprising at least one silicon atom, and especially at least one Si-O group.

The term "hydrocarbon-based oil" means an oil comprising mainly hydrogen and carbon atoms.

The term "fluoro oil" means an oil comprising at least one fluorine atom.

A hydrocarbon-based oil that is suitable for use in the invention may also optionally comprise oxygen, nitrogen, sulfur and/or phosphorus atoms, for example in the form of hydroxyl, amine, amide, ester, ether or acid groups, and in particular in the form of hydroxyl, ester, ether or acid groups.

The oily phase generally comprises, in addition to the lipophilic UV-screening agent or agents, at least one volatile or non-volatile hydrocarbon-based oil and/or one volatile and/or non-volatile silicone oil.

For the purposes of the invention, the term "volatile oil" means an oil that is capable of evaporating on contact with the skin or the keratin fibre in less than one hour, at room temperature and atmospheric pressure. The volatile oil(s) of the invention are volatile cosmetic oils which are liquid at room temperature and which have a non-zero vapour pressure, at room temperature and atmospheric pressure, ranging in particular from 0.13 Pa to 40 000 Pa ($10^{-3}$ to 300 mmHg), in particular ranging from 1.3 Pa to 13 000 Pa (0.01 to 100 mmHg) and more particularly ranging from 1.3 Pa to 1300 Pa (0.01 to 10 mmHg).

The term "non-volatile oil" means an oil that remains on the skin or the keratin fibre at room temperature and atmospheric pressure for at least several hours, and that especially has a vapour pressure of less than $10^{-3}$ mmHg (0.13 Pa).

### Hydrocarbon-based oils

As non-volatile hydrocarbon-based oils that may be used according to the invention, mention may be made especially of:

(i) hydrocarbon-based oils of plant origin, such as glyceride triesters, which are generally triesters of fatty acids and of glycerol, the fatty acids of which can have varied chain lengths from $C_4$ to $C_{24}$, it being possible for these chains to be saturated or unsaturated and linear or branched; these oils are in particular wheatgerm oil, sunflower oil, grape seed oil, sesame oil, corn oil, apricot oil, castor oil, shea oil, avocado oil, olive oil, soybean oil, sweet almond oil, palm oil, rapeseed oil, cottonseed oil, hazelnut oil, macadamia oil, jojoba oil, alfalfa oil, poppy oil, pumpkin oil, marrow

oil, blackcurrant oil, evening primrose oil, millet oil, barley oil, quinoa oil, rye oil, safflower oil, candlenut oil, passionflower oil and musk rose oil; or also caprylic/capric acid triglycerides, such as those sold by Stéarineries Dubois or those sold under the names Miglyol 810®, 812® and 818® by Dynamit Nobel,

(ii) synthetic ethers containing from 10 to 40 carbon atoms;

(iii) linear or branched hydrocarbons of mineral or synthetic origin, such as petroleum jelly, polydecenes, hydrogenated polyisobutene such as Parleam, and squalane, and mixtures thereof;

(iv) synthetic esters, for instance oils of formula RCOOR' in which R represents a linear or branched fatty acid residue containing from 1 to 40 carbon atoms and R' represents a hydrocarbon-based chain that is especially branched, containing from 1 to 40 carbon atoms, on condition that R + R' is ≥10, for instance purcellin oil (cetostearyl octanoate), isopropyl myristate, isopropyl palmitate, $C_{12}$-$C_{15}$ alkyl benzoate, such as the product sold under the trade name Finsolv TN® or Witconol TN® by the company Witco or Tegosoft TN® by the company Evonik Goldschmidt, 2-ethylphenyl benzoate, such as the commercial product sold under the name X-Tend 226® by the company ISP, isopropyl lanolate, hexyl laurate, diisopropyl adipate, isononyl isononanoate, oleyl erucate, 2-ethylhexyl palmitate, isostearyl isostearate, diisopropyl sebacate, such as the product sold under the name of "Dub Dis" by the company Stéarineries Dubois, octanoates, decanoates or ricinoleates of alcohols or polyalcohols, such as propylene glycol dioctanoate; hydroxylated esters, such as isostearyl lactate or diisostearyl malate; and pentaerythritol esters; citrates or tartrates, such as di(linear $C_{12}$-$C_{13}$ alkyl) tartrates, such as those sold under the name Cosmacol ETI® by the company Enichem Augusta Industriale, and also di(linear $C_{14}$-$C_{15}$ alkyl) tartrates, such as those sold under the name Cosmacol ETL® by the same company; or acetates;

(v) fatty alcohols that are liquid at room temperature, containing a branched and/or unsaturated carbon-based chain containing from 12 to 26 carbon atoms, for instance octyldodecanol, isostearyl alcohol, oleyl alcohol, 2-hexyldecanol, 2-butyloctanol or 2-undecylpentadecanol;

(vi) higher fatty acids, such as oleic acid, linoleic acid or linolenic acid;

(vii) carbonates, such as dicaprylyl carbonate, such as the product sold under the name Cetiol CC® by the company Cognis;

(viii) fatty amides, such as isopropyl N-lauroyl sarcosinate, such as the product sold under the trade name Eldew SL205® from Ajinomoto;

and mixtures thereof.

Among the non-volatile hydrocarbon-based oils that may be used according to the invention, preference will be given more particularly to glyceride triesters and in particular to caprylic/capric acid triglycerides, synthetic esters and in particular isononyl isononanoate, oleyl erucate, $C_{12}$-$C_{15}$ alkyl benzoate, 2-ethylphenyl benzoate and fatty alcohols, in particular octyldodecanol.

As volatile hydrocarbon-based oils that may be used according to the invention, mention may be made especially of hydrocarbon-based oils having from 8 to 16 carbon atoms and in particular of branched $C_8$-$C_{16}$ alkanes, such as $C_8$-$C_{16}$ isoalkanes of petroleum origin (also known as isoparaffins), such as isododecane (also known as 2,2,4,4,6-pentamethylheptane), isodecane or isohexadecane, the oils sold under the Isopar or Permethyl trade names, branched $C_8$-$C_{16}$ esters, isohexyl neopentanoate, and mixtures thereof.

Mention may also be made of the alkanes described in the Cognis patent applications WO 2007/068 371 or WO 2008/155 059 (mixtures of distinct alkanes differing by at least one carbon). These alkanes are obtained from fatty alcohols, which are themselves obtained from coconut or palm oil. Mention may be made of the mixtures of n-undecane ($C_{11}$) and n-tridecane ($C_{13}$) obtained in Examples 1 and 2 of patent application WO 2008/155 059 from the company Cognis. Mention may also be made of n-dodecane ($C_{12}$) and n-tetradecane ($C_{14}$) sold by Sasol under the respective references Parafol 12-97 and Parafol 14-97®, and also mixtures thereof.

Other volatile hydrocarbon-based oils, for instance petroleum distillates, especially those sold under the name Shell Solt® by the company Shell, may also be used. According to one embodiment, the volatile solvent is chosen from volatile hydrocarbon-based oils containing from 8 to 16 carbon atoms, and mixtures thereof.

b) <u>Silicone oils</u>

The non-volatile silicone oils may be chosen in particular from non-volatile polydimethylsiloxanes (PDMSs),

polydimethylsiloxanes comprising alkyl or alkoxy groups which are pendent and/or at the end of the silicone chain, which groups each contain from 2 to 24 carbon atoms, or phenyl silicones, such as phenyl trimethicones, phenyl dimethicones, phenyl(trimethylsiloxy)diphenylsiloxanes, diphenyl dimethicones, diphenyl(methyldiphenyl)trisiloxanes or (2-phenylethyl)trimethylsiloxysilicates.

Examples of volatile silicone oils that may be mentioned include volatile linear or cyclic silicones, especially those with a viscosity ≤ 8 centistokes ($8 \times 10^{-6}$ $m^2/s$) and especially containing from 2 to 7 silicon atoms, these silicones optionally comprising alkyl or alkoxy groups containing from 1 to 10 carbon atoms. As volatile silicone oils that may be used in the invention, mention may be made especially of octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane and dodecamethylpentasiloxane, and mixtures thereof.

Mention may also be made of the volatile linear alkyltrisiloxane oils of general formula (I):

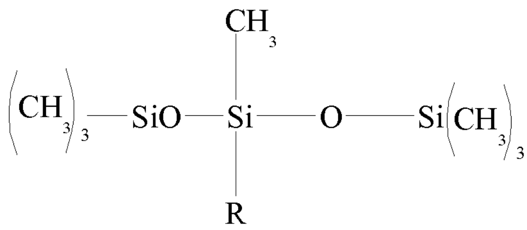

where R represents an alkyl group comprising from 2 to 4 carbon atoms, one or more hydrogen atoms of which may be replaced with a fluorine or chlorine atom.

Among the oils of general formula (I), mention may be made of:

3-butyl-1,1,1,3,5,5,5-heptamethyltrisiloxane,
3-propyl-1,1,1,3,5,5,5-heptamethyltrisiloxane, and
3-ethyl-1,1,1,3,5,5,5-heptamethyltrisiloxane,
corresponding to the oils of formula (I) for which R is, respectively, a butyl group, a propyl group or an ethyl group.

Fluoro oils

Use may also be made of volatile fluoro oils, such as nonafluoromethoxybutane, decafluoropentane, tetradecafluorohexane, dodecafluoropentane, and mixtures thereof.

An oily phase according to the invention may also comprise other fatty substances, mixed with or dissolved in the oil.

Another fatty substance that may be present in the oily phase may be, for example:

- a fatty acid chosen from fatty acids comprising from 8 to 30 carbon atoms, such as stearic acid, lauric acid, palmitic acid and oleic acid;
- a wax chosen from waxes such as lanolin, beeswax, carnauba or candelilla wax, paraffin waxes, lignite waxes, microcrystalline waxes, ceresin or ozokerite, or synthetic waxes, such as polyethylene waxes or Fischer-Tropsch waxes;
- a gum chosen from silicone gums (dimethiconol);
- a pasty compound, such as polymeric or non-polymeric silicone compounds, esters of a glycerol oligomer, arachidyl propionate, fatty acid triglycerides and derivatives thereof;
- and mixtures thereof.

Preferably, the overall oily phase, including all the lipophilic substances of the composition capable of being dissolved in this same phase, represents from 5% to 95% by weight and preferably from 10% to 80% by weight, with respect to the total weight of the composition.

**AQUEOUS PHASE**

The compositions according to the invention comprise at least one aqueous phase.

The aqueous phase comprises water and optionally other water-soluble or water-miscible organic solvents.

An aqueous phase that is suitable for use in the invention may comprise, for example, a water chosen from a natural spring water, such as water from La Roche-Posay, water from Vittel or waters from Vichy, or a floral water.

The water-soluble or water-miscible solvents that are suitable for use in the invention comprise short-chain monoalcohols, for example $C_1$-$C_4$ monoalcohols, such as ethanol or isopropanol; diols or polyols, such as ethylene glycol, 1,2-propylene glycol, 1,3-butylene glycol, hexylene glycol, diethylene glycol, dipropylene glycol, 2-ethoxyethanol, diethylene glycol monomethyl ether, triethylene glycol monomethyl ether, glycerol and sorbitol, and mixtures thereof.
According to a preferred embodiment, use may more particularly be made of ethanol, propylene glycol, glycerol, and mixtures thereof.
According to a particular form of the invention, the overall aqueous phase, including all the hydrophilic substances of the composition capable of being dissolved in this same phase, represents from 5% to 95% by weight and preferably from 10% to 80% by weight, relative to the total weight of the composition.

## ADDITIVES

### a) Additional UV-screening agents

The compositions according to the invention may also contain one or more additional UV-screening agents chosen from hydrophilic, lipophilic or insoluble organic UV-screening agents and/or one or more mineral pigments. It will preferentially consist of at least one hydrophilic, lipophilic or insoluble organic UV-screening agent.
The additional organic UV-screening agents are chosen in particular from cinnamic compounds; anthranilate compounds; salicylic compounds; dibenzoylmethane compounds; benzylidenecamphor compounds; benzophenone compounds; β,β-diphenylacrylate compounds; triazine compounds; benzotriazole compounds; benzalmalonate compounds, in particular those cited in patent US 5 624 663; benzimidazole derivatives; imidazoline compounds; bis-benzazolyl compounds, as described in patents EP 669 323 and US 2 463 264; p-aminobenzoic acid (PABA) compounds; methylenebis(hydroxyphenylbenzotriazole) compounds, as described in patent applications US 5 237 071, US 5 166 355, GB 2 303 549, DE 197 26 184 and EP 893 119; benzoxazole compounds, as described in patent applications EP 0 832 642, EP 1 027 883, EP 1 300 137 and DE 101 62 844; screening polymers and screening silicones, such as those described in particular in patent application WO 93/04665; α-alkylstyrene-based dimers, such as those described in patent application DE 198 55 649; 4,4-diarylbutadiene compounds, as described in patent applications EP 0 967 200, DE 197 46 654, DE 197 55 649, EP-A-1 008 586, EP 1 133 980 and EP 133 981, and mixtures thereof.
As examples of organic photoprotective agents, mention may be made of those denoted hereinbelow under their INCI name:

Cinnamic compounds:

Ethylhexyl methoxycinnamate sold especially under the trade name Parsol MCX® by DSM Nutritional Products,

Isopropyl methoxycinnamate,
Isoamyl p-methoxycinnamate sold under the trade name Neo Heliopan E 1000® by Symrise,
DEA Methoxycinnamate,
Diisopropyl methyl cinnamate,
Glyceryl ethylhexanoate dimethoxycinnamate.

Dibenzoylmethane compounds:

Butylmethoxydibenzoylmethane sold in particular under the trade name Parsol 1789® by DSM Nutritional Products, Inc.;
Isopropyldibenzoylmethane.

para-Aminobenzoic compounds:

PABA,
Ethyl PABA,
Ethyl dihydroxypropyl PABA,
Ethylhexyl dimethyl PABA, sold in particular under the name Escalol 507® by ISP, Glyceryl PABA,
PEG-25 PABA, sold under the name Uvinul P 25® by BASF.

Salicylic compounds:

Homosalate, sold under the name Eusolex HMS® by Rona/EM Industries,
Ethylhexyl salicylate, sold under the name Neo Heliopan OS® by Symrise,
Dipropylene glycol salicylate, sold under the name Dipsal® by Scher,
TEA salicylate, sold under the name Neo Heliopan TS® by Symrise.

β,β-Diphenylacrylate compounds:

Octocrylene, sold in particular under the trade name Uvinul N 539® by BASF,
Etocrylene, sold in particular under the trade name Uvinul N 35® by BASF.

Benzophenone compounds:

Benzophenone-1, sold under the trade name Uvinul 400® by BASF,
Benzophenone-2, sold under the trade name Uvinul 50® by BASF,
Benzophenone-3 or Oxybenzone, sold under the trade name Uvinul M 40® by BASF,
Benzophenone-4, sold under the trade name Uvinul MS 40® by BASF, Benzophenone-5,
Benzophenone-6, sold under the trade name Helisorb 11® by Norquay,
Benzophenone-8, sold under the trade name Spectra-Sorb UV-24® by American Cyanamid,
Benzophenone-9, sold under the trade name Uvinul DS 49® by BASF, Benzophenone-12,
n-Hexyl 2-(4-diethylamino-2-hydroxybenzoyl)benzoate, sold under the trade name Uvinul A Plus® or, as a mixture with octyl methoxycinnamate, under the trade name Uvinul A Plus B® by BASF,
1,1'-(1,4-piperazinediyl)bis[1-[2-[4-(diethylamino)-2-hydroxybenzoyl]phenyl]-methanone] (CAS 919803-06-8), such as described in patent application WO 2007/071 584; this compound advantageously being used in micronized form (mean size of 0.02 to 2 μm), which may be obtained, for example, according to the micronization process described in patent applications GB-A-2 303 549 and EP-A-893 119, and in particular in the form of an aqueous dispersion.

Benzylidenecamphor compounds:

3-Benzylidenecamphor, manufactured under the name Mexoryl SD® by Chimex, 4-Methylbenzylidenecamphor, sold under the name Eusolex 6300® by Merck,
Benzylidenecamphorsulfonic acid, manufactured under the name Mexoryl SL® by Chimex,
Camphor benzalkonium methosulfate, manufactured under the name Mexoryl SO® by Chimex,
Terephthalylidenedicamphorsulfonic acid, manufactured under the name Mexoryl SX® by Chimex,
Polyacrylamidomethylbenzylidenecamphor, manufactured under the name Mexoryl SW® by Chimex.

Phenylbenzimidazole compounds:

Phenylbenzimidazolesulfonic acid, sold in particular under the trade name Eusolex 232® by Merck.

Bis-benzazolyl compounds:

Disodium phenyl dibenzimidazole tetrasulfonate, sold under the trade name Neo Heliopan AP® by Haarmann and Reimer.

Phenylbenzotriazole compounds:

Drometrizole trisiloxane, sold under the name Silatrizole® by Rhodia Chimie.
Methylenebis(hydroxyphenylbenzotriazole) compounds:

Methylenebis(benzotriazolyl)tetramethylbutylphenol especially in solid form, for instance the product sold under the trade name Mixxim BB/100® by Fairmount Chemical, or in the form of an aqueous dispersion of micronized particles with a mean particle size ranging from 0.01 to 5 μm, more preferentially from 0.01 to 2 μm and more particularly from 0.020 to 2 μm, with at least one alkylpolyglycoside surfactant having the structure $C_nH_{2n+1}O(C_6H_{10}O_5)_xH$, in which n is an integer from 8 to 16 and x is the mean degree of polymerization of the $(C_6H_{10}O_5)$ unit and ranges from 1.4 to 1.6, as described in patent GB-A-2 303 549, sold especially under the trade name Tinosorb M® by BASF, or in the form of an aqueous dispersion of micronized particles with a mean particle size ranging from 0.02 to 2 μm, more preferentially from 0.01 to 1.5 μm and more particularly from 0.02 to 1 μm, in the presence of at least one polyglyceryl mono($C_8$-$C_{20}$)alkyl ester with a degree of glycerol polymerization of at least 5, such as the aqueous dispersions described in patent application WO 2009/063 392.

Triazine compounds:

- Bis(ethylhexyloxyphenol)methoxyphenyltriazine, sold under the trade name Tinosorb S® by BASF,
- Ethylhexyl Triazone, sold in particular under the trade name Uvinul T150® by BASF,
- Diethylhexyl Butamido Triazone, sold under the trade name Uvasorb HEB® by Sigma 3V,
- 2,4,6-tris(dineopentyl 4'-aminobenzalmalonate)-s-triazine,
- 2,4,6-tris(diisobutyl 4'-aminobenzalmalonate)-s-triazine;
- 2,4-bis(n-butyl 4'-aminobenzoate)-6-(aminopropyltrisiloxane)-s-triazine,
- 2,4-bis(dineopentyl 4'-aminobenzalmalonate)-6-(n-butyl 4'-aminobenzoate)-s-triazine,
- symmetrical triazine screening agents substituted with naphthalenyl groups or polyphenyl groups described in patent US 6 225 467, patent application WO 2004/085 412 (see compounds 6 and 9) or the document "Symmetrical Triazine Derivatives", IP.COM IPCOM000031257 Journal, INC, West Henrietta, NY, US (20 September 2004), in particular 2,4,6-tris(diphenyl)triazine and 2,4,6-tris(terphenyl)triazine, which is also mentioned in patent applications WO 06/035 000, WO 06/034 982, WO 06/034 991, WO 06/035 007, WO 2006/034 992 and WO 2006/034 985, these compounds advantageously being used in micronized form (mean particle size of 0.02 to 3 μm), which may be obtained, for example, according to the micronization process described in patent applications GB-A-2 303 549 and EP-A-893 119, and in particular in the form of an aqueous dispersion;
- silicone triazines substituted with two aminobenzoate groups, as described in patent EP 0 841 341, in particular 2,4-bis(n-butyl 4'-aminobenzalmalonate)-6-[(3-{1,3,3,3-tetramethyl-1-[(trimethylsilyl)oxy]disiloxanyl}propyl)amino]-s-triazine.

Anthranilic compounds:

Menthyl anthranilate, sold under the trade name Neo Heliopan MA® by Symrise.

Imidazoline compounds:

Ethylhexyl dimethoxybenzylidene dioxoimidazoline propionate.

Benzalmalonate compounds:

Polyorganosiloxane comprising benzalmalonate functional groups, such as Polysilicone-15, sold under the trade name Parsol SLX® by Hoffmann-LaRoche.

4,4-Diarylbutadiene compounds:

1,1-Dicarboxy(2,2'-dimethylpropyl)-4,4-diphenylbutadiene.

Benzoxazole compounds:

2,4-Bis[4-[5-(1,1-dimethylpropyl)benzoxazol-2-yl]phenylimino]-6-[(2-ethylhexyl)imino]-1,3,5-triazine, sold under the name of Uvasorb K2A® by Sigma 3V.

The preferred organic screening agents are chosen from:

Ethylhexyl methoxycinnamate,

Ethylhexyl salicylate,
Homosalate,
Butylmethoxydibenzoylmethane,
Octocrylene,
Phenylbenzimidazolesulfonic acid,
Benzophenone-3,
Benzophenone-4,
Benzophenone-5,
n-Hexyl 2-(4-diethylamino-2-hydroxybenzoyl)benzoate,
4-Methylbenzylidenecamphor,
Terephthalylidenedicamphorsulfonic acid,
Disodium phenyl dibenzimidazole tetrasulfonate,
Methylenebis(benzotriazolyl)tetramethylbutylphenol,
Bis(ethylhexyloxyphenol)methoxyphenyltriazine,
Ethylhexyl Triazone,
Diethylhexyl Butamido Triazone,
2,4,6-Tris(dineopentyl 4'-aminobenzalmalonate)-s-triazine
2,4,6-Tris(diisobutyl 4'-aminobenzalmalonate)-s-triazine,
2,4-Bis(n-butyl 4'-aminobenzoate)-6-(aminopropyltrisiloxane)-s-triazine,
2,4-Bis(dineopentyl 4'-aminobenzalmalonate)-6-(n-butyl 4'-aminobenzoate)-s-triazine,
2,4-Bis(n-butyl 4'-aminobenzalmalonate)-6-[(3-{1,3,3,3-tetramethyl-1-[(trimethylsilyl)oxy]disiloxanyl}propyl)amino]-s-triazine,
2,4,6-Tris(diphenyl)triazine,
2,4,6-Tris(terphenyl)triazine,
Drometrizole Trisiloxane,
Polysilicone-15,
1,1-Dicarboxy(2,2'-dimethylpropyl)-4,4-diphenylbutadiene,
2,4-Bis[4-[5-(1,1-dimethylpropyl)benzoxazol-2-yl]phenylimino]-6-[(2-ethylhexyl)imino]-1,3,5-triazine,
and mixtures thereof.

The particularly preferred organic screening agents are chosen from:

Ethylhexyl salicylate,
Homosalate,
Butylmethoxydibenzoylmethane,
Octocrylene,
n-Hexyl 2-(4-diethylamino-2-hydroxybenzoyl)benzoate,
Terephthalylidene Dicamphor Sulfonic Acid,
Bis(ethylhexyloxyphenol)methoxyphenyltriazine,
Ethylhexyl Triazone,
Diethylhexyl Butamido Triazone,
2,4-Bis(n-butyl 4'-aminobenzalmalonate)-6-[(3-{1,3,3,3-tetramethyl-1-[(trimethylsilyl)oxy]disiloxanyl}propyl)amino]-s-triazine,
Drometrizole Trisiloxane,
and mixtures thereof.

The mineral UV-screening agents used in accordance with the present invention are metal oxide pigments. More preferentially, the mineral UV-screening agents of the invention are metal oxide particles with a mean elementary particle size of less than or equal to 0.5 μm, more preferentially between 0.005 and 0.5 μm, even more preferentially between 0.01 and 0.2 μm, better still between 0.01 and 0.1 μm and more particularly between 0.015 and 0.05 μm.

They may be selected in particular from titanium oxide, zinc oxide, iron oxide, zirconium oxide and cerium oxide, or mixtures thereof.

Such coated or uncoated metal oxide pigments are described in particular in patent application EP-A-0 518 773. Commercial pigments that may be mentioned include the products sold by the companies Sachtleben Pigments, Tayca, Merck and Degussa.

The metal oxide pigments may be coated or uncoated.

The coated pigments are pigments that have undergone one or more surface treatments of chemical, electronic, mechanochemical and/or mechanical nature with compounds such as amino acids, beeswax, fatty acids, fatty alcohols,

anionic surfactants, lecithins, sodium, potassium, zinc, iron or aluminium salts of fatty acids, metal alkoxides (of titanium or aluminium), polyethylene, silicones, proteins (collagen, elastin), alkanolamines, silicon oxides, metal oxides or sodium hexametaphosphate.

The coated pigments are more particularly titanium oxides that have been coated:

- with silica, such as the product Sunveil® from the company Ikeda,
- with silica and iron oxide, such as the product Sunveil F® from the company Ikeda,
- with silica and alumina, such as the products Microtitanium Dioxide MT 500 SA® and Microtitanium Dioxide MT 100 SA® from the company Tayca, and Tioveil from the company Tioxide,
- with alumina, such as the products Tipaque TTO-55 (B)® and Tipaque TTO-55 (A)® from the company Ishihara and UVT 14/4 from the company Sachtleben Pigments,
- with alumina and aluminium stearate, such as the products Microtitanium Dioxide MT 100 T®, MT 100 TX®, MT 100 Z® and MT-01® from the company Tayca, the products Solaveil CT-10 W® and Solaveil CT 100® from the company Uniqema and the product Eusolex T-AVO® from the company Merck,
- with silica, alumina and alginic acid, such as the product MT-100 AQ® from the company Tayca,
- with alumina and aluminium laurate, such as the product Microtitanium Dioxide MT 100 S® from the company Tayca,
- with iron oxide and iron stearate, such as the product Microtitanium Dioxide MT 100 F® from the company Tayca,
- with zinc oxide and zinc stearate, such as the product BR 351® from the company Tayca,
- with silica and alumina and treated with a silicone, such as the products Microtitanium Dioxide MT 600 SAS®, Microtitanium Dioxide MT 500 SAS® or Microtitanium Dioxide MT 100 SAS® from the company Tayca,
- with silica, alumina and aluminium stearate and treated with a silicone, such as the product STT-30-DS® from the company Titan Kogyo,
- with silica and treated with a silicone, such as the product UV-Titan X 195® from the company Sachtleben Pigments,
- with alumina and treated with a silicone, such as the products Tipaque TTO-55 (S)® from the company Ishihara or UV Titan M 262® from the company Sachtleben Pigments,
- with triethanolamine, such as the product STT-65-S from the company Titan Kogyo,
- with stearic acid, such as the product Tipaque TTO-55 (C)® from the company Ishihara,
- with sodium hexametaphosphate, such as the product Microtitanium Dioxide MT 150 W® from the company Tayca,
- $TiO_2$ treated with octyltrimethylsilane, sold under the trade name T 805® by the company Degussa Silices,
- $TiO_2$ treated with a polydimethylsiloxane, sold under the trade name 70250 Cardre UF TiO2SI3® by the company Cardre,
- anatase/rutile $TiO_2$ treated with a polydimethylhydrosiloxane, sold under the trade name Microtitanium Dioxide USP Grade Hydrophobic® by the company Color Techniques.

Mention may also be made of $TiO_2$ pigments doped with at least one transition metal such as iron, zinc or manganese and more particularly manganese. Preferably, the said doped pigments are in the form of an oily dispersion. The oil present in the oily dispersion is preferably chosen from triglycerides including those of capric/caprylic acids. The oily dispersion of titanium oxide particles may also comprise one or more dispersants, for instance a sorbitan ester, for instance sorbitan isostearate, or a polyoxyalkylenated fatty acid ester of glycerol, for instance TRI-PPG3 myristyl ether citrate and polyglyceryl-3 polyricinoleate. Preferably, the oily dispersion of titanium oxide particles comprises at least one dispersant chosen from polyoxyalkylenated fatty acid esters of glycerol. Mention may be made more particularly of the oily dispersion of $TiO_2$ particles doped with manganese in capric/caprylic acid triglycerides in the presence of TRI-PPG-3 myristyl ether citrate and polyglyceryl-3 polyricinoleate and sorbitan isostearate having the INCI name: titanium dioxide (and) TRI-PPG-3 myristyl ether citrate (and) polyglyceryl-3 ricinoleate (and) sorbitan isostearate, for instance the product sold under the trade name Optisol TD50® by the company Croda.

The uncoated titanium oxide pigments are sold, for example, by the company Tayca under the trade names Microtitanium Dioxide MT 500 B or Microtitanium Dioxide MT 600 B®, by the company Degussa under the name P 25, by the company Wackher under the name Transparent Titanium Oxide PW®, by the company Miyoshi Kasei under the name UFTR®, by the company Tomen under the name ITS® and by the company Tioxide under the name Tioveil AQ®.

The uncoated zinc oxide pigments are for example:

- those sold under the name Z-Cote by the company Sunsmart;
- those sold under the name Nanox® by the company Elementis;
- those sold under the name Nanogard WCD 2025® by the company Nanophase Technologies.

The coated zinc oxide pigments are for example:

- those sold under the name Zinc Oxide CS-5® by the company Toshibi (ZnO coated with polymethylhydrogenosiloxane);

- those sold under the name Nanogard Zinc Oxide FN® by the company Nanophase Technologies (as a 40% dispersion in Finsolv TN®, $C_{12}$-$C_{15}$ alkyl benzoate);
- those sold under the name Daitopersion Zn-30® and Daitopersion Zn-50® by the company Daito (dispersions in cyclopolymethylsiloxane/oxyethylenated polydimethylsiloxane, containing 30% or 50% of zinc oxides coated with silica and polymethylhydrogenosiloxane);
- those sold under the name NFD Ultrafine ZnO® by the company Daikin (ZnO coated with perfluoroalkyl phosphate and copolymer based on perfluoroalkylethyl as a dispersion in cyclopentasiloxane);
- those sold under the name SPD-Z1® by the company Shin-Etsu (ZnO coated with silicone-grafted acrylic polymer, dispersed in cyclodimethylsiloxane);
- those sold under the name Escalol Z100® by the company ISP (alumina-treated ZnO dispersed in an ethylhexyl methoxycinnamate/PVP-hexadecene copolymer/methicone mixture);
- those sold under the name Fuji ZnO-SMS-10® by the company Fuji Pigment (ZnO coated with silica and polymethylsilsesquioxane);
- those sold under the name Nanox Gel TN® by the company Elementis (ZnO dispersed at a concentration of 55% in $C_{12}$-$C_{15}$ alkyl benzoate with hydroxystearic acid polycondensate).

The uncoated cerium oxide pigments may be, for example, those sold under the name Colloidal Cerium Oxide® by the company Rhone-Poulenc.

The uncoated iron oxide pigments are sold, for example, by the company Arnaud under the names Nanogard WCD 2002® (FE 45B®), Nanogard Iron FE 45 BL AQ®, Nanogard FE 45R AQ® and Nanogard WCD 2006® (FE 45R®) or by the company Mitsubishi under the name TY-220®.

The coated iron oxide pigments are sold, for example, by the company Arnaud under the names Nanogard WCD 2008 (FE 45B FN)®, Nanogard WCD 2009® (FE 45B 556®), Nanogard FE 45 BL 345® and Nanogard FE 45 BL® or by the company BASF under the name Transparent Iron Oxide®.

Mention may also be made of mixtures of metal oxides, in particular of titanium dioxide and of cerium dioxide, including the equal-weight mixture of titanium dioxide and cerium dioxide coated with silica, sold by the company Ikeda under the name Sunveil A®, and also the mixture of titanium dioxide and zinc dioxide coated with alumina, silica and silicone, such as the product M 261® sold by the company Sachtleben Pigments, or coated with alumina, silica and glycerol, such as the product M 211® sold by the company Sachtleben Pigments.

According to the invention, coated or uncoated titanium oxide pigments are particularly preferred.

The additional UV-screening agents according to the invention are preferably present in the compositions according to the invention in a content ranging from 0.1 % to 45% by weight and in particular from 5% to 30% by weight relative to the total weight of the composition.

**b) <u>Other additives:</u>**

The aqueous compositions in accordance with the present invention may also comprise conventional cosmetic adjuvants chosen in particular from organic solvents, ionic or nonionic thickeners, softeners, humectants, opacifiers, stabilizers, emollients, silicones, antifoams, fragrances, preserving agents, anionic, cationic, nonionic, zwitterionic or amphoteric surfactants, active agents, fillers, polymers, propellants, basifying or acidifying agents or any other ingredient commonly used in the cosmetic and/or dermatological field.

Among the organic solvents that may be mentioned are lower alcohols and polyols. These polyols may be chosen from glycols and glycol ethers, for instance ethylene glycol, propylene glycol, butylene glycol, dipropylene glycol or diethylene glycol.

Thickeners that may be mentioned include carboxyvinyl polymers such as the Carbopol products (carbomers) and the Pemulen products (acrylate/$C_{10}$-$C_{30}$-alkyl acrylate copolymer) (PemulenTR1® or PemulenTR2®); polyacrylamides, for instance the crosslinked copolymers sold under the names Sepigel 305® (CTFA name: polyacrylamide/$C_{13-14}$ isoparaffin/Laureth 7) or Simulgel 600 (CTFA name: acrylamide/sodium acryloyldimethyl taurate copolymer/isohexadecane/polysorbate 80) by the company SEPPIC; 2-acrylamido-2-methylpropanesulfonic acid polymers and copolymers, optionally crosslinked and/or neutralized, such as the poly(2-acrylamido-2-methylpropanesulfonic acid) sold by the company Hoechst under the trade name Hostacerin AMPS® (CTFA name: ammonium polyacryloyldimethyl taurate) or Simulgel 800®, sold by the company SEPPIC (CTFA name: sodium polyacryloyldimethyl taurate/polysorbate 80/sorbitan oleate); copolymers of 2-acrylamido-2-methylpropanesulfonic acid and of hydroxyethyl acrylate, such as Simulgel NS® and Sepinov EMT 10®, sold by the company SEPPIC; cellulose derivatives, such as hydroxyethylcellulose; polysaccharides and in particular gums, such as xanthan gum; water-soluble or water-dispersible silicone derivatives, such as acrylic silicones, polyether silicones and cationic silicones, and mixtures thereof.

Among the acidifying agents, examples that may be mentioned include mineral or organic acids, for instance

hydrochloric acid, orthophosphoric acid, sulfuric acid, carboxylic acids, for instance acetic acid, tartaric acid, citric acid or lactic acid, and sulfonic acids.

Among the basifying agents, examples that may be mentioned include aqueous ammonia, alkali metal carbonates, alkanolamines, such as mono-, di- and triethanolamines and derivatives thereof, sodium hydroxide or potassium hydroxide.

Preferably, the cosmetic composition comprises one or more basifying agents selected from alkanolamines, in particular triethanolamine, and sodium hydroxide.

In the case of a direct emulsion, the pH of the composition in accordance with the invention is generally between 3 and 12 approximately, preferably between 5 and 11 approximately and even more particularly from 6 to 8.5.

Among the active agents for caring for keratin materials such as the skin, the lips, the scalp, the hair, the eyelashes or the nails, examples that may be mentioned include:

- vitamins and derivatives or precursors thereof, alone or as mixtures;
- antioxidants;
- free-radical scavengers;
- antipollution agents;
- self-tanning agents;
- antiglycation agents;
- calmatives;
- deodorants;
- essential oils;
- NO-synthase inhibitors;
- agents for stimulating the synthesis of dermal or epidermal macromolecules and/or for preventing their degradation,
- agents for stimulating fibroblast proliferation;
- agents for stimulating keratinocyte proliferation;
- muscle relaxants,
- refreshing agents;
- tensioning agents;
- mattifying agents;
- depigmenting agents;
- propigmenting agents;
- keratolytic agents;
- desquamating agents;
- moisturizers;
- antiinflammatory agents;
- antimicrobial agents;
- slimming agents;
- agents acting on the energy metabolism of cells;
- insect repellents;
- substance P or CGRP antagonists;
- hair-loss counteractants;
- antiwrinkle agents;
- antiageing agents.

A person skilled in the art will select the said active agent(s) as a function of the effect desired on the skin, the hair, the eyelashes, the eyebrows and the nails.

Needless to say, a person skilled in the art will take care to select the abovementioned optional additional compound(s) and/or the amounts thereof such that the advantageous properties intrinsically associated with the compositions in accordance with the invention are not, or are not substantially, adversely affected by the envisaged addition(s).

## GALENICAL FORMS

The compositions according to the invention may be prepared according to the techniques that are well known to those skilled in the art for manufacturing oil-in-water emulsions or water-in-oil emulsions, and in particular oil-in-water emulsions. They may be in the form of a cream, a milk or a cream gel. They may optionally be in the form of a mousse or a spray.

The compositions according to the invention are preferably in the form of an oil-in-water emulsion.

The emulsification processes that may be used are of the paddle or propeller, rotor-stator and HPH type.

In order to obtain stable emulsions with a low content of polymer (oil/polymer ratio > 25), it is possible to prepare the dispersion in concentrated phase and then to dilute the dispersion with the remainder of the aqueous phase.

It is also possible, by means of an HPH (between 50 and 800 bar), to obtain stable dispersions with drop sizes that may be as low as 100 nm.

The aqueous phase of the emulsions may comprise a nonionic vesicular dispersion prepared according to known processes (Bangham, Standish and Watkins, J. Mol. Biol., 13, 238 (1965), FR 2 315 991 and FR 2 416 008).

The compositions according to the invention find their application in a large number of treatments, especially cosmetic treatments, of the skin, the lips and the hair, including the scalp, especially for protecting and/or caring for the skin, the lips and/or the hair, and/or for making up the skin and/or the lips.

Another subject of the present invention consists of the use of the compositions according to the invention as defined above for the manufacture of products for cosmetic treatment of the skin, the lips, the nails, the hair, the eyelashes, the eyebrows and/or the scalp, especially care products, antisun products and makeup products.

The cosmetic compositions according to the invention may be used, for example, as makeup products.

Another subject of the present invention consists of a non-therapeutic cosmetic process for caring for and/or making up a keratin material, which consists in applying, to the surface of the said keratin material, at least one composition according to the invention as defined above.

The cosmetic compositions according to the invention may be used, for example, as care products and/or antisun products for the face and/or body with a liquid to semi-liquid consistency, such as milks, more or less smooth creams, cream gels or pastes. They may optionally be packaged in aerosol form and may be in the form of a mousse or a spray.

The compositions according to the invention in the form of vaporizable fluid lotions in accordance with the invention are applied to the skin or the hair in the form of fine particles by means of pressurization devices. The devices in accordance with the invention are well known to those skilled in the art and comprise non-aerosol pumps or "atomizers", aerosol containers comprising a propellant and aerosol pumps using compressed air as propellant. These devices are described in patents US 4 077 441 and US 4 850 517.

The compositions packaged in aerosol form in accordance with the invention generally contain conventional propellants, for instance hydrofluoro compounds, dichlorodifluoromethane, difluoroethane, dimethyl ether, isobutane, n-butane, propane or trichlorofluoromethane. They are preferably present in amounts ranging from 15% to 50% by weight relative to the total weight of the composition.

**ASSEMBLY**

According to another aspect, the invention also relates to a cosmetic assembly comprising:

i) a container delimiting one or more compartment(s), the said container being closed by a closing member and optionally not being leaktight; and
ii) a makeup and/or care composition in accordance with the invention placed inside the said compartment(s).

The container may be, for example, in the form of a jar or a box.

The closing member may be in the form of a lid comprising a cap mounted so as to be able to move by translation or by pivoting relative to the container housing the said makeup and/or care composition(s).

The examples that follow serve to illustrate the invention without, however, being limiting in nature. In these examples, the amounts of the composition ingredients are given as weight percentages relative to the total weight of the composition.

**Example A1: Preparation of compound (1)**

(1) 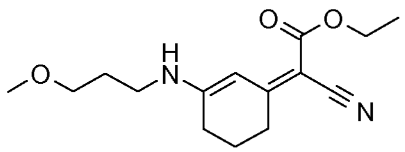

122.23 g of 3-[(3-methoxypropyl)amino]-2-cyclohexen-1-one were alkylated with dimethyl sulfate or alternatively with diethyl sulfate and treated with 75.45 g of ethyl cyanoacetate in approximately equimolar proportions in the presence of a base and optionally of a solvent. The following base/solvent combinations were used:

| Example | Base | Solvent |
|---|---|---|
| Example A1.1 | DBU (1,8-diazabicyclo[5.4.0]undec-7-ene) | dimethylacetamide |
| Example A1.2 | triethylamine | isopropanol |
| Example A1.3 | 3-methoxypropylamine | isopropanol |
| Example A1.4 | 3-methoxypropylamine | tert-amyl alcohol |
| Example A1.5 | 3-methoxypropylamine | toluene |
| Example A1.6 | 3-methoxypropylamine | dimethylformamide |
| Example A1.7 | 3-methoxypropylamine | no solvent |
| Example A1.8 | N-morpholine | isopropanol |

The completion of the alkylation reaction was monitored, for example, via methods such as TLC, GC or HPLC. 162.30 g of compound (14) were obtained in the form of a brown oil. After crystallization, the product was obtained in the form of yellowish crystals. Melting point: 92.7°C.

**Example A2: Preparation of compound (2)**

(2)

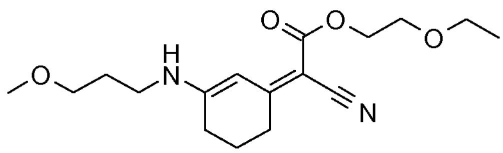

148.4 g of 3-[(3-methoxypropyl)amino]-2-cyclohexen-1-one were alkylated with dimethyl sulfate or alternatively with diethyl sulfate and treated with 130.00 g of 2-ethoxyethyl cyanoacetate in the presence of an organic base and a solvent. The following base/solvent combinations were used:

| Example | Base | Solvent |
|---|---|---|
| Example A2.1 | DBU (1,8-diazabicyclo[5.4.0]undec-7-ene) | dimethylacetamide |
| Example A2.2 | triethylamine | isopropanol |
| Example A2.3 | 3-methoxypropylamine | isopropanol |
| Example A2.4 | N-methylmorpholine | tert-amyl alcohol |
| Example A2.5 | 3-methoxypropylamine | toluene |
| Example A2.6 | 3-methoxypropylamine | dimethylformamide |
| Example A2.7 | 3-methoxypropylamine | No solvent |

**Example A3 (outside the invention): Preparation of the compound (2Z)-2-cyano-N-(3-methoxypropyl)-2-{3-[(3-methoxypropyl)amino]cyclohex-2-en-1-ylidene}ethanamide described in the unpublished patent application PCT/EP 2012/064 195**

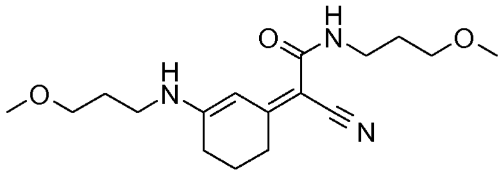

101.00 g of 3-[(3-methoxypropyl)amino]-2-cyclohexen-1-one were alkylated with dimethyl sulfate or alternatively with diethyl sulfate and treated with 86.00 g of 2-cyano-N-(3-methoxypropyl)acetamide in approximately equimolar proportions in the presence of a base and optionally of a solvent.

The following base/solvent combinations were used:

| Example | Base | Solvent |
|---|---|---|
| Example A3.1 | DBU (1,8-diazabicyclo[5.4.0]undec-7-ene) | dimethylacetamide |
| Example A3.2 | triethylamine | isopropanol |
| Example A3.3 | 3-methoxypropylamine | isopropanol |
| Example A3.4 | 3-methoxypropylamine | tert-amyl alcohol |
| Example A3.5 | 3-methoxypropylamine | toluene |
| Example A3.6 | 3-methoxypropylamine | dimethylformamide |
| Example A3.7 | 3-methoxypropylamine | no solvent |

The crude product (2Z)-2-cyano-N-(3-methoxypropyl)-2-{3-[(3-methoxypropyl)amino]cyclohex-2-en-1-ylidene} ethanamide was obtained in the form of a dark brown oil. After chromatography on a column of silica gel (eluent: 99/1 toluene/methanol), 81.8 g of product were obtained in the form of yellowish crystals.
Melting point: 84.7-85.3°C.

## Formulation Examples 1 to 4

Formulations 1 to 4 were prepared.

| Phase | Ingredients | Formulation 1 (invention) | Formulation 2 (invention) |
|---|---|---|---|
| A | Water | qsp 100 | qsp 100 |
| | Glycerol | 5 | 5 |
| | Triethanolamine | 0.45 | 0.45 |
| | Disodium EDTA | 0.1 | 0.1 |
| | Ammonium acryloyldimethyltaurate/steareth -8 methacrylate copolymer (Aristoflex SNC®) | 0.5 | 0.5 |
| | Ammonium polyacryldimethyltauramide (Hostacerin AMPS® from Clariant) | 0.4 | 0.4 |
| | Xanthan gum | 0.2 | 0.2 |
| B | 2-Ethylphenyl benzoate (X-Tend 226®) | 30 | 30 |
| | Compound (2) | 0.5 | 2 |
| | Glyceryl stearate | 0.5 | 0.5 |

| | | | |
|---|---|---|---|
| | Alcohol | 1 | 1 |
| | Preserving agents | 1 | 1 |
| | Isohexadecane | 1 | 1 |

| Phase | Ingredients | Formulation 3 (outside the invention) | Formulation 4 (outside the invention) |
|---|---|---|---|
| A | Water | qsp 100 | qsp 100 |
| | Glycerol | 5 | 5 |
| | Triethanolamine | 0.65 | 0.65 |
| | Disodium EDTA | 0.1 | 0.1 |
| | Potassium cetyl phosphate (Amphisol K®) | 1 | 1 |
| | Acrylates/C10-30 alkyl acrylate crosspolymer (Pemulen TR1®) | 0.2 | 0.2 |
| | Xanthan gum | 0.2 | 0.2 |
| B | 2-Ethylphenyl benzoate (X-Tend 226®) | 30 | 30 |
| | Compound (2) | 0.5 | 2 |
| | Stearic acid | 1.5 | 1.5 |
| | Glyceryl stearate (and) PEG-100 stearate (Arlacel 165®) | 2.5 | 2.5 |
| | Cetyl alcohol | 0.5 | 0.5 |
| | Cetearyl alcohol (and) cetearyl glucoside (Montanov 68®) | 2 | 2 |
| | Dimethicone | 0.5 | 0.5 |
| | Preserving agents | 1 | 1 |
| | Isohexadecane | 1 | 1 |

**Emulsion preparation method:**

The aqueous phase A and oily phase B were prepared by mixing the starting materials with mechanical stirring at 80°C. Once the aqueous solution A and oily solution B were macroscopically homogeneous, the emulsion was prepared by introducing phase B into phase A with stirring using a rotor-stator homogenizer at a stirring speed of 4500 rpm for 20 minutes. The emulsion was cooled to room temperature. The final emulsion was characterized by drops of between 1 µm and 20 µm in size.

**Protocol for evaluating the merocyanine stability:**

The stability of the merocyanines in formulation was evaluated after storage of the formulations for 2 months. It was established by UPLC assay of the residual merocyanine content after 2 months at 45°C relative to the residual

content after 2 months at 4°C. The percentage of merocyanine degradation is expressed as:

$$\text{Degradation}_{t2M}\,(\%) = \frac{\text{Merocyanine content}_{t2M4°C} - \text{Merocyanine content}_{t2M45°C}}{\text{Merocyanine content}_{t2M4°C}} \times 100$$

| Evaluated chemical stability | Formulation 1 (invention) | Formulation 2 (invention) | Formulation 3 (outside the invention) | Formulation 4 (outside the invention) |
|---|---|---|---|---|
| Merocyanine degradation at $t_{2M45°C}$ versus $t_{2M\ 4°C}$ (%) | <1% | <1% | 7.5% | 11.3% |

The chemical stability results for the merocyanine compounds in formulations 1 and 2 of the invention are better than those measured for formulations 3 and 4. This results demonstrates that the emulsifying systems comprising at least one amphiphilic polymer comprising at least one 2-acrylamidomethylpropanesulfonic acid unit ensure better chemical stability of the merocyanines of the invention.

**Formulation Examples 5 and 6**

The following formulations were prepared:

The compound 2-ethoxyethyl (2Z)-cyano{3-[(3-methoxypropyl)amino]cyclohex-2-en-1-ylidene}ethanoate (2) of the invention was compared with: the compound (2Z)-2-cyano-N-(3-methoxy-propyl)-2-{3-[(3-methoxypropyl)amino]cyclohex-2-en-1-ylidene}ethanamide according to Example A3 (outside the invention) the compound octyl-5-N,N-diethylamino-2-phenysulfonyl-2,4-pentadienoate (outside the invention).

The *in vitro* SPF and the *in vitro* $UVA_{PPD}$ index of each formulation were also measured.

| Phase | Ingredients | Formulation 5 (invention) | Formulation 6 (outside the invention) |
|---|---|---|---|
| A | Water | qsp 100 | qsp 100 |
| | Mixture of glycols:<br>Glycerol 6%<br>Glycol 2%<br>Pentylene glycol 3% | 11 | 11 |
| | Disodium EDTA | 0.1 | 0.1 |
| | Triethanolamine | 0.4 | 0.4 |
| | Terephthalylidenedicamphorsulfonic acid (Mexoryl SX®) | 0.75 | 0.75 |
| | Styrene/acrylate copolymer (Sunspheres Powder® - Röhm & Haas) | 3 | 3 |
| | Ammonium acryloyldimethyltaurate/steareth-8 methacrylate copolymer (Aristoflex SNC®) | 0.5 | 0.5 |
| | Ammonium polyacryldimethyltauramide (Hostacerin AMPS® from Clariant) | 0.4 | 0.4 |
| | Xanthan gum | 0.2 | 0.2 |
| B | 2-Ethylphenyl benzoate (X-Tend 226®) | 11.5 | 11.5 |
| | Isopropyl lauroyl sarcosinate (Eldew SL-205®) | 0.5 | 0.5 |
| | Caprylic/capric triglyceride | 2.5 | 2.5 |
| | Compound (2) | 0.63 | - |
| | (2Z)-2-cyano-N-(3-methoxypropyl)-2-{3-[(3-methoxypropyl)amino]cyclohex-2-en-1-ylidene}ethanamide | - | 0.77 |
| | Butylmethoxydibenzoylmethane (Avobenzone) | 3 | 3 |
| | Bis(ethylhexyloxyphenol)methoxyphenyltriazine (Tinosorb S®) | 4.5 | 4.5 |
| | Drometrizole Trisiloxane, (Mexoryl XL®) | 1 | 1 |
| | Ethylhexyl Triazone | 2 | 2 |

| | | | |
|---|---|---|---|
| | (Uvinul T150®) | | |
| | Titanium dioxide (and) aluminium hydroxide (and) stearic acid (Microtitanium dioxide MT-100 TV® - Tayca) | 5 | 5 |
| | Synthetic Wax (Polyethylene wax - Cirebelle 303) | 1 | 1 |
| | Stearyl alcohol | 1 | 1 |
| | Glyceryl isostearate | 0.5 | 0.5 |
| | Antioxidant | 0.5 | 0.5 |
| | Preserving agents | 0.9 | 0.9 |
| **C** | Nylon + Talc | 2 | 2 |
| | Alcohol | 5 | 5 |

| **Phase** | **Ingredients** | **Formulation 7 (invention)** | **Formulation 8 (outside the invention)** |
|---|---|---|---|
| A | Water | qs 100 | qs 100 |
| | Glycerol | 5.0 | 5.0 |
| | Disodium EDTA | 0.1 | 0.1 |
| | Phenylbenzimidazolesulfonic acid (Eusolex 232® from Merck) | 1.0 | 1.0 |
| | Triethanolamine | 0.75 | 0.75 |
| | Disodium stearoyl glutamate (Amisoft HS 21P® from Ajinomoto) | 0.5 | 0.5 |
| | Polyacrylamide (and) $C_{13}$-$C_{14}$ isoparaffin (and) laureth-7 (Sepigel 305® from SEPPIC) | 1.3 | 1.3 |
| B | 2-Ethylphenyl benzoate (X-Tend 226) | 5.55 | 5.55 |
| | Compound (2) | 0.63 | - |
| | (2Z)-2-cyano-N-(3-methoxypropyl)-2-{3-[(3-methoxypropyl)amino]cyclohex-2-en-1-ylidene}ethanamide | - | 0.77 |
| | Octocrylene | 2.0 | 2.0 |
| | Butylmethoxydibenzoylmethane | 2.0 | 2.0 |
| | Ethylhexyl salicylate | 4.0 | 4.0 |
| | Titanium dioxide (and) stearic acid (and) alumina (UV Titan M 160® - | 0.5 | 0.5 |

| | | | |
|---|---|---|---|
| | Sachtleben) | | |
| | Antioxidant | 0.2 | 0.2 |
| | Preserving agents | 1.05 | 1.05 |
| | Cetyl alcohol (Lanette 16® from BASF) | 0.5 | 0.5 |
| | Behenyl alcohol (Lanette 22® from BASF) | 1.0 | 1.0 |
| | PEG-100 stearate (Myrj S100-PA-(SG)® from Croda) | 0.3 | 0.3 |
| | Cetearyl alcohol (and) cetearyl glucoside (Montanov 68®) | 0.32 | 0.32 |
| | Carbomer (Synthalen K® from 3V) | 0.07 | 0.07 |
| | Ammonium acryloyldimethyltaurate/steareth-25 methacrylate crosspolymer (Aristoflex HMS®) | 0.5 | 0.5 |
| C | Polytetrafluoroethylene wax (Ceridust 9205 F® from Clariant) | 0.3 | 0.3 |
| | Alcohol | 2.0 | 2.0 |

**Emulsion preparation method:**

The aqueous phase A and oily phase B were prepared by mixing the starting materials with mechanical stirring at 80°C. Once the aqueous solution A and oily solution B were macroscopically homogeneous, the emulsion was prepared by introducing phase B into phase A with stirring using a rotor-stator homogenizer at a stirring speed of 4500 rpm for 20 minutes. The emulsion was cooled to room temperature before adding, one by one, the ingredients of phase C. The final emulsion was characterized by drops between 1 μm and 20 μm in size.

**Protocol for evaluating the merocyanine stability:**

The stability of the merocyanines in formulation was evaluated after storage of the formulations for 2 months. It was established by UPLC assay of the residual merocyanine content after 2 months at 45°C relative to the residual content after 2 months at 4°C. The percentage of merocyanine degradation is expressed as:

$$\text{Degradation}_{t2M}\,(\%) = \frac{\text{Merocyanine content}_{t2M4°C} - \text{Merocyanine content}_{t2M45°C}}{\text{Merocyanine content}_{t2M4°C}} \times 100$$

***in vitro* protocol for evaluating the screening efficacy**

The sun protection factor (SPF) was determined according to the *in vitro* method described by B.L. Diffey in J. Soc. Cosmet. Chem. 40, 127-133 (1989). The measurements were carried out using a UV-1000S spectrophotometer from the company Labsphere. The "static *in vitro* protection factor (SPF)" value is extracted. Each composition is applied to a rough plate of PMMA in the form of a uniform and even deposit in a proportion of 1 mg/cm$^2$. The *in vitro* PPD index measurements are taken under the same conditions using a UV-1000S spectrophotometer from the company Labsphere. The "UV-$A_{ppd}$ index (persistent pigment darkening action spectrum)" value is extracted. Each composition is applied to a rough plate of PMMA, in the form of a uniform and even deposit in a proportion of 1 mg/cm$^2$.

| Measurements obtained | Formulation 5 (invention) | Formulation 6 (outside the invention) | Formulation 7 (invention) | Formulation 8 (outside the invention) |
|---|---|---|---|---|
| ***in vitro* SPF** | 164 ± 23 | 162 ± 25 | 14.9 ± 1.0 | 16.8 ± 1.2 |
| ***in vitro* $UVA_{PPD}$** | 111 ± 18 | 112 ± 18 | 12.1 ± 0.7 | 13.6 ± 0.9 |
| **Merocyanine degradation at $t_{2M\ 45°C}$ versus $t_{2M\ 4°C}$ (%)** | 4% | 36% | 4% | 40% |

The stability results show that the merocyanine of the invention (compound (2)), formulated in the emulsified compositions 5 and 7 of the invention, is chemically more stable than the merocyanine outside the invention (2Z)-2-cyano-N-(3-methoxypropyl)-2-{3-[(3-methoxypropyl)amino]cyclohex-2-en-1-ylidene}ethanamide in the emulsified compositions 6 and 8.

## Claims

1. Cosmetic or dermatological composition in emulsion form, comprising, in a physiologically acceptable support:

a) at least one aqueous phase, and
b) at least one oily phase, and
c) at least one merocyanine compound of formula (1) or one of the E/E- or E/Z-geometrical isomer forms thereof:

(1) 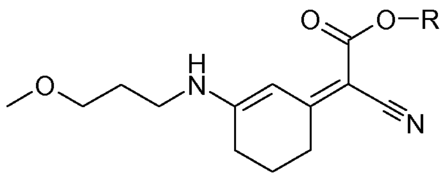

in which:

R is a $C_1$-$C_{22}$ alkyl group, a $C_2$-$C_{22}$ alkenyl group, a $C_2$-$C_{22}$ alkynyl group, a $C_3$-$C_{22}$ cycloalkyl group or a $C_3$-$C_{22}$ cycloalkenyl group, the said groups possibly being interrupted with one or more O, and

d) at least one emulsifying system containing at least one amphiphilic polymer comprising at least one 2-acrylamidomethylpropanesulfonic acid unit.

2. Composition according to Claim 1, in which the merocyanine compound(s) are chosen from those in which R is a $C_1$-$C_{22}$ alkyl, which may be interrupted with one or more O.

3. Composition according to Claim 1 or 2, in which the merocyanine compound(s) are chosen from the following compounds, and also the E/E- or E/Z- geometrical isomer forms thereof:

| | | | |
|---|---|---|---|
| 1 | 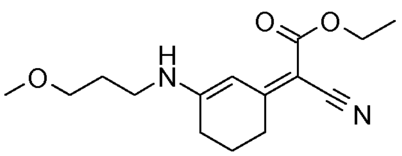 ethyl (2Z)-cyano{3-[(3-methoxypropyl)amino]cyclohex-2-en-1-ylidene} ethanoate | 4 | 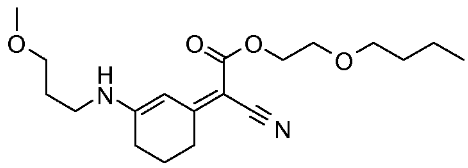 2-butoxyethyl (2Z)-cyano{3-[(3-methoxypropyl)amino]cyclohex-2-en-1-ylidene} ethanoate |

(continued)

| | | | |
|---|---|---|---|
| 2 | 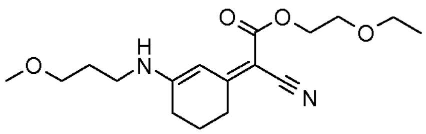<br>2-ethoxyethyl (2Z)-cyano{3-[(3-methoxypropyl)amino]cyclohex-2-en-1-ylidene} ethanoate | 5 | 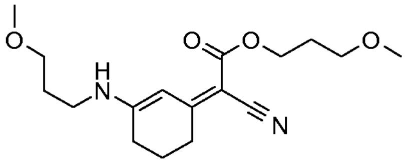<br>3-methoxypropyl (2Z)-cyano{3-[(3-methoxypropyl)amino]cyclohex-2-en-1-ylidene} ethanoate |
| 3 | 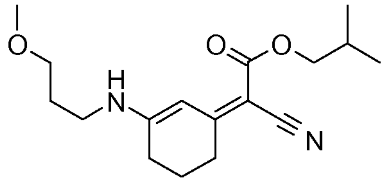<br>2-methylpropyl (2Z)-cyano{3-[(3-methoxypropyl)amino]cyclohex-2-en-1-ylidene} ethanoate | 6 | 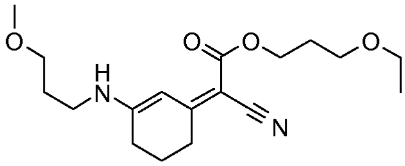<br>3-ethoxypropyl (2Z)-cyano{3-[(3-methoxypropyl)amino]cyclohex-2-en-1-ylidene} ethanoate |

**4.** Composition according to Claim 3, in which the merocyanine compound is 2-ethoxyethyl (2Z)-cyano{3-[(3-methoxypropyl)amino]cyclohex-2-en-1-ylidenelethanoate (2) in its E/Z geometrical configuration having the following structure:

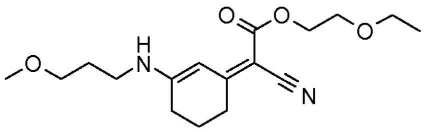

and/or in its E/E geometrical configuration having the following structure:

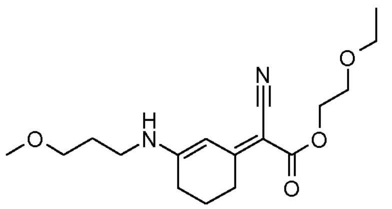

**5.** Composition according to any one of Claims 1 to 4, in which the merocyanine compound(s) of formula (1) are present in a concentration ranging from 0.1% to 10% by weight and preferentially from 0.2% to 5% by weight relative to the total weight of the composition.

**6.** Composition according to any one of Claims 1 to 4, in which the amphiphilic polymer comprising at least one 2-acrylamidomethylpropanesulfonic acid unit is a crosslinked or non-crosslinked polymer consisting of:

a) 2-acrylamido-2-methylpropanesulfonic acid (AMPS®) units of formula (II) below:

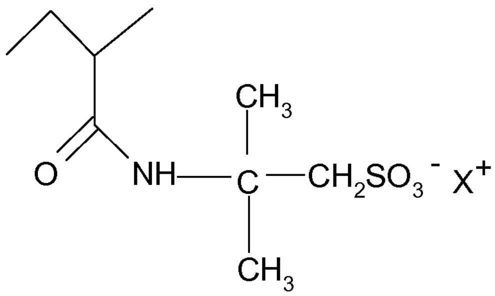

(II)

in which X is a proton, an alkali metal cation, an alkaline-earth metal cation or an ammonium ion; and
b) units of formula (III) below:

(III)

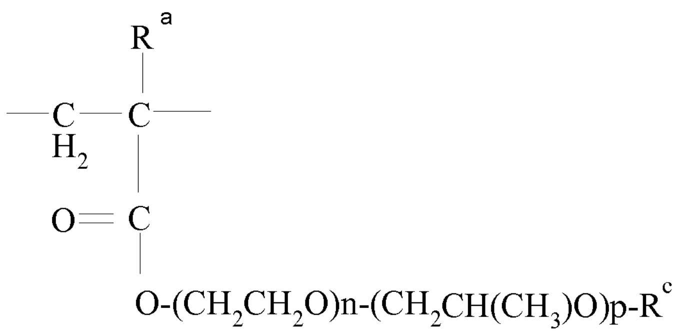

in which n and p, independently of one another, denote a number of moles and range from 0 to 30, preferably from 1 to 25 and more preferentially from 3 to 20, with the proviso that n + p is less than or equal to 30, preferably less than 25 and better still less than 20; $R^a$ denotes a hydrogen atom or a linear or branched $C_1$-$C_6$ alkyl radical, preferably methyl, and $R^c$ denotes a linear or branched alkyl containing from 7 to 22 carbon atoms and preferably from 12 to 22 carbon atoms.

**7.** Composition according to Claim 6, in which, in formula (II), the cation X denotes sodium or ammonium.

**8.** Composition according to Claim 6 or 7, in which the monomer of formula (III) is chosen from:

- esters of (meth)acrylic acid and of a $C_{10}$-$C_{18}$ fatty alcohol polyoxyethylenated with 8 mol of ethylene oxide,
- esters of (meth)acrylic acid and of a $C_{11}$ fatty oxoalcohol polyoxyethylenated with 8 mol of ethylene oxide,
- esters of (meth)acrylic acid and of a $C_{12}$-$C_{14}$ fatty alcohol polyoxyethylenated with 7 mol of ethylene oxide,
- esters of (meth)acrylic acid and of a $C_{12}$-$C_{14}$ fatty alcohol polyoxyethylenated with 11 mol of ethylene oxide,
- esters of (meth)acrylic acid and of a $C_{16}$-$C_{18}$ fatty alcohol polyoxyethylenated with 8 mol of ethylene oxide,
- esters of (meth)acrylic acid and of a $C_{16}$-$C_{18}$ fatty alcohol polyoxyethylenated with 15 mol of ethylene oxide,
- esters of (meth)acrylic acid and of a $C_{16}$-$C_{18}$ fatty alcohol polyoxyethylenated with 11 mol of ethylene oxide,
- esters of (meth)acrylic acid and of a $C_{16}$-$C_{18}$ fatty alcohol polyoxyethylenated with 20 mol of ethylene oxide,
- esters of (meth)acrylic acid and of a $C_{16}$-$C_{18}$ fatty alcohol polyoxyethylenated with 25 mol of ethylene oxide,
- esters of (meth)acrylic acid and of a $C_{18}$-$C_{22}$ fatty alcohol polyoxyethylenated with 25 mol of ethylene oxide and/or of a $C_{16}$-$C_{18}$ fatty isoalcohol polyoxyethylenated with 25 mol of ethylene oxide.

**9.** Composition according to any one of Claims 6 to 8, in which the amphiphilic polymer comprising at least one 2-acrylamidomethylpropanesulfonic acid unit is chosen from:

- the non-crosslinked products for which p = 0, n = 7 or 25, $R^a$ denotes a methyl and $R^c$ represents a mixture of $C_{12}$-$C_{14}$ or $C_{16}$-$C_{18}$ alkyl,
- the crosslinked products for which p = 0, n = 8 or 25, $R^a$ denotes a methyl and $R^c$ represents a mixture of $C_{16}$-$C_{18}$ alkyl.

**10.** Composition according to any one of Claims 6 to 9, in which the amphiphilic polymer comprising at least one 2-acrylamidomethylpropanesulfonic acid unit is chosen from:

- a non-crosslinked copolymer of 2-acrylamidomethylpropanesulfonic acid and of a $C_{12}$-$C_{14}$ alkyl methacrylate polyethoxylated with 25 mol of ethylene oxide (Ammonium acryloyldimethyltaurate/laureth-7 methacrylate copolymer),
- a crosslinked copolymer of 2-acrylamidomethylpropanesulfonic acid and of stearyl methacrylate polyethoxylated with 25 mol of ethylene oxide (Ammonium acryloyldimethyltaurate/steareth-25 methacrylate crosspolymer),
- a non-crosslinked copolymer of 2-acrylamidomethylpropanesulfonic acid and of a $C_{16}$/$C_{18}$ alkyl methacrylate polyethoxylated with 8 mol of ethylene oxide (Ammonium acryloyldimethyltaurate/steareth-8 methacrylate copolymer),
- a crosslinked copolymer of 2-acrylamidomethylpropanesulfonic acid and of behenyl methacrylate polyethoxylated with 25 mol of ethylene oxide (Ammonium acryloyldimethyltaurate/beheneth-25 methacrylate crosspolymer), and mixtures thereof.

**11.** Composition according to any one of Claims 6 to 10, in which the amphiphilic polymer comprising at least one 2-acrylamidomethylpropanesulfonic acid unit is a copolymer of 2-acrylamidomethylpropanesulfonic acid and of a $C_{16}$-$C_{18}$ alkyl methacrylate comprising from 6 to 25 mol of ethylene oxide.

**12.** Composition according to Claim 11, in which the amphiphilic polymer comprising at least one 2-acrylamidomethylpropanesulfonic acid unit is a copolymer of 2-acrylamidomethylpropanesulfonic acid and of a $C_{16}$-$C_{18}$ alkyl methacrylate comprising from 6 to 10 mol of ethylene oxide, for which the mole proportion of $C_{16}$-$C_{18}$ alkyl methacrylate units (units (II)) ranges from 2% to 15% and better still from 5% to 10%, and even more particularly a copolymer of 2-acrylamidomethylpropanesulfonic acid and of an ammonium salt of methacrylic acid and of oxyethylenated stearyl alcohol comprising 8 mol of ethylene oxide and comprising a mole proportion of alkyl methacrylate units of 7.35% (Ammonium acryloyldimethyltaurate/steareth-8 methacrylate copolymer).

**13.** Composition according to any one of the preceding claims, in which the composition is in the form of an oil-in-water emulsion or a water-in-oil emulsion and more particularly in the form of an oil-in-water emulsion.

**14.** Non-therapeutic cosmetic process for caring for and/or making up a keratin material, comprising the application, to the surface of the said keratin material, of at least one composition as defined in any one of the preceding claims.

**15.** Non-therapeutic cosmetic process for limiting the darkening of the skin and/or improving the colour and/or the uniformity of the complexion, comprising the application, to the surface of the skin, of at least one composition as defined in any one of the preceding claims.

**16.** Non-therapeutic cosmetic process for preventing and/or treating the signs of ageing of a keratin material, comprising the application, to the surface of the keratin material, of at least one composition as defined in any one of the preceding claims.

**Patentansprüche**

**1.** Kosmetische oder dermatologische Zusammensetzung in Emulsionsform, umfassend in einem physiologisch unbedenklichen Träger:

a) mindestens eine wässrige Phase und
b) mindestens eine ölige Phase und
c) mindestens eine Merocyanin-Verbindung der Formel (1) oder eine der geometrischen E/E- oder E/Z-Isomerformen davon:

(1)

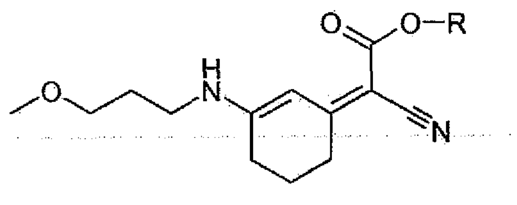

wobei:

R für eine $C_1$-$C_{22}$-Alkylgruppe, eine $C_2$-$C_{22}$-Alkenylgruppe, eine $C_2$-$C_{22}$-Alkinylgruppe, eine $C_3$-$C_{22}$-Cycloalkylgruppe oder eine $C_3$-$C_{22}$-Cycloalkenyl-gruppe steht, wobei die Gruppen gegebenenfalls durch ein oder mehrere O unterbrochen sein können, und

d) mindestens ein Emulgiersystem, das mindestens ein amphiphiles Polymer mit mindestens einer 2-Acrylamidomethylpropansulfonsäure-Einheit enthält.

**2.** Zusammensetzung nach Anspruch 1, wobei die Merocyanin-Verbindung(en) aus desjenigen ausgewählt sind, in denen R für ein $C_1$-$C_{22}$-Alkyl, das durch ein oder mehrere O unterbrochen sein kann, steht.

**3.** Zusammensetzung nach Anspruch 1 oder 2, wobei die Merocyanin-Verbindung(en) aus den folgenden Verbindungen sowie den geometrischen E/E- oder E/Z-Isomerformen davon ausgewählt sind:

| | | | |
|---|---|---|---|
| 1 | 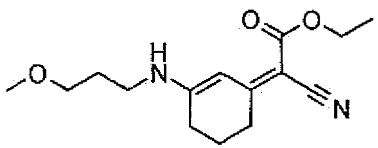 (2Z)-Cyano{3-[(3-methoxypropyl)amino]-cyclohex-2-en-1-yliden}ethansäure-ethylester | 4 | 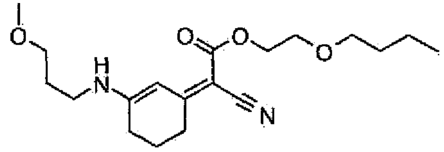 (2Z)-Cyano{3-[(3-methoxypropyl)amino]-cyclohex-2-en-1-yliden}ethansäure-2-butoxyethylester |
| 2 | 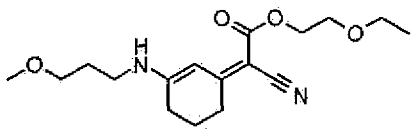 (2Z)-Cyano{3-[(3-methoxypropyl)amino]-cyclohex-2-en-1-yliden}ethansäure-2-ethoxyethylester | 5 | 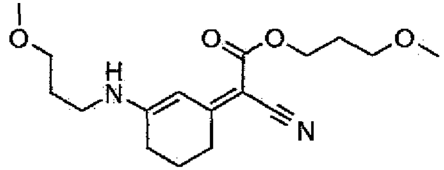 (2Z)-Cyano{3-[(3-methoxypropyl)amino]-cyclohex-2-en-1-yliden}ethansäure-3-methoxypropylester |
| 3 | 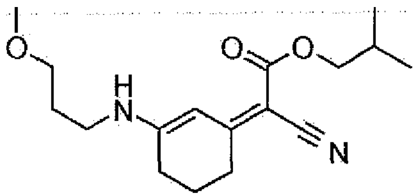 (2Z)-Cyano{3-[(3-methoxypropyl)amino]-cyclohex-2-en-1-yliden}ethansäure-2-methylpropylester | 6 | 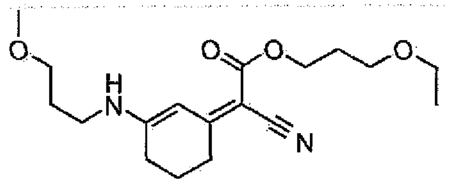 (2Z)-Cyano{3-[(3-methoxypropyl)amino]-cyclohex-2-en-1-yliden}ethansäure-3-ethoxypropylester |

4. Zusammensetzung nach Anspruch 3, wobei es sich bei der Merocyanin-Verbindung um (2Z)-Cyano{3-[(3-methoxypropyl)amino]cyclohex-2-en-1-yliden}ethan-säure-2-ethoxyethylester (2) in seiner geometrischen E/Z-Konfiguration mit der folgenden Struktur:

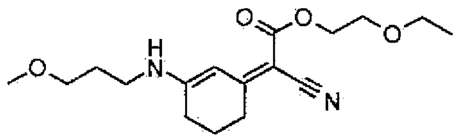

und/oder in seiner geometrischen E/E-Konfiguration mit der folgenden Struktur:

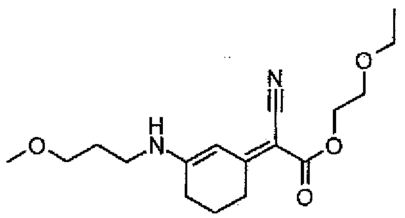

handelt.

5. Zusammensetzung nach einem der Ansprüche 1 bis 4, wobei die Merocyanin-Verbindung(en) der Formel (1) in einer Konzentration im Bereich von 0,1 bis 10 Gew.-% und vorzugsweise von 0,2 bis 5 Gew.-%, bezogen auf das Gesamtgewicht der Zusammensetzung, vorliegt bzw. vorliegen.

6. Zusammensetzung nach einem der Ansprüche 1 bis 4, wobei es sich bei dem amphiphilen Polymer mit mindestens einer 2-Acrylamidomethylpropansulfonsäure-Einheit um ein vernetztes oder unvernetztes Polymer aus:

a) 2-Acrylamido-2-methylpropansulfonsäure(AMPS®)-Einheiten der nachstehenden Formel (II):

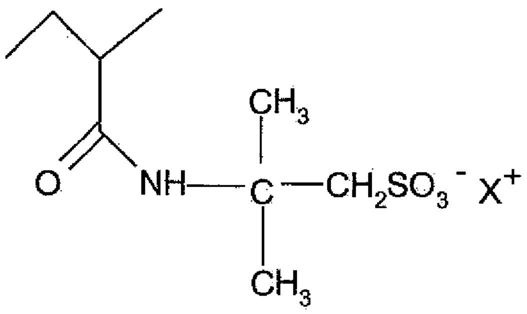

(II)

wobei X für ein Proton, ein Alkalimetallkation, ein Erdalkalimetallkation oder ein Ammoniumion steht; und
b) Einheiten der nachstehenden Formel (III):

(III)

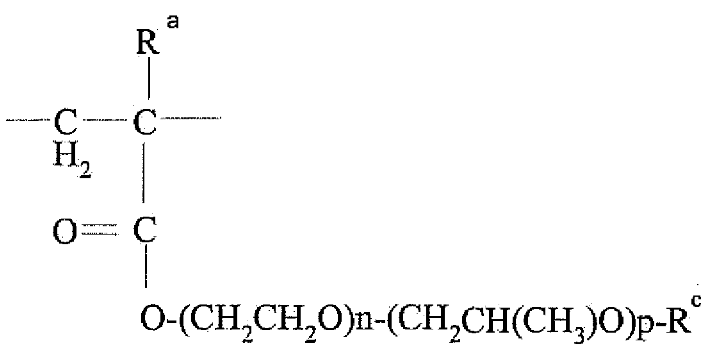

wobei n und p unabhängig voneinander für eine Molzahl stehen und im Bereich von 0 bis 30, vorzugsweise 1

bis 25 und weiter bevorzugt 3 bis 20 liegen, mit der Maßgabe, dass n + p kleiner gleich 30, vorzugsweise kleiner als 25 und noch besser kleiner als 20 ist; $R^a$ für ein Wasserstoffatom oder einen linearen oder verzweigten $C_1$-$C_6$-Alkylrest, vorzugsweise Methyl, steht und $R^c$ für ein lineares oder verzweigtes Alkyl mit 7 bis 22 Kohlenstoffatomen und vorzugsweise 12 bis 22 Kohlenstoffatomen steht; handelt.

**7.** Zusammensetzung nach Anspruch 6, wobei in Formel (II) das Kation X für Natrium oder Ammonium steht.

**8.** Zusammensetzung nach Anspruch 6 oder 7, wobei das Monomer der Formel (III) aus:

- Estern von (Meth)acrylsäure und einem mit 8 mol Ethylenoxid polyoxyethylenierten $C_{10}$-$C_{18}$-Fett-alkohol,
- Estern von (Meth)acrylsäure und einem mit 8 mol Ethylenoxid polyoxyethylenierten $C_{11}$-Fettoxo-alkohol,
- Estern von (Meth)acrylsäure und einem mit 7 mol Ethylenoxid polyoxyethylenierten $C_{12}$-$C_{14}$-Fett-alkohol,
- Estern von (Meth)acrylsäure und einem mit 11 mol Ethylenoxid polyoxyethylenierten $C_{12}$-$C_{14}$-Fett-alkohol,
- Estern von (Meth)acrylsäure und einem mit 8 mol Ethylenoxid polyoxyethylenierten $C_{16}$-$C_{18}$-Fett-alkohol,
- Estern von (Meth)acrylsäure und einem mit 15 mol Ethylenoxid polyoxyethylenierten $C_{16}$-$C_{18}$-Fettalkohol,
- Estern von (Meth)acrylsäure und einem mit 11 mol Ethylenoxid polyoxyethylenierten $C_{16}$-$C_{18}$-Fett-alkohol,
- Estern von (Meth)acrylsäure und einem mit 20 mol Ethylenoxid polyoxyethylenierten $C_{16}$-$C_{18}$-Fettalkohol,
- Estern von (Meth)acrylsäure und einem mit 25 mol Ethylenoxid polyoxyethylenierten $C_{16}$-$C_{18}$-Fettalkohol,
- Estern von (Meth)acrylsäure und einem mit 25 mol Ethylenoxid polyoxyethylenierten $C_{18}$-$C_{22}$-Fettalkohol und/oder einem mit 25 Mol Ethylenoxid polyoxyethylenierten $C_{16}$-$C_{18}$-Fettisoalkohol ausgewählt ist.

**9.** Zusammensetzung nach einem der Ansprüche 6 bis 8, wobei das amphiphile Polymer mit mindestens einer 2-Acrylamidomethylpropansulfonsäure-Einheit aus:

- den unvernetzten Produkten, für die p = 0, n = 7 oder 25, $R^a$ für Methyl steht und $R^c$ für ein Gemisch von $C_{12}$-$C_{14}$- oder $C_{16}$-$C_{18}$-Alkyl steht,
- den vernetzten Produkten, für die p = 0, n = 8 oder 25, $R^a$ für Methyl steht und $R^c$ für ein Gemisch von $C_{16}$-$C_{18}$-Alkyl steht,

ausgewählt ist.

**10.** Zusammensetzung nach einem der Ansprüche 6 bis 9, wobei das amphiphile Polymer mit mindestens einer 2-Acrylamidomethylpropansulfonsäure-Einheit aus:

- einem unvernetzten Copolymer von 2-Acrylamidomethylpropansulfonsäure und einem mit 25 mol Ethylenoxid polyethoxylierten $C_{12}$-$C_{14}$-Alkylmethacrylat (Ammoniumacryloyldimethyltaurat/Laureth-7-methacrylat-Copolymer),
- einem vernetzten Copolymer von 2-Acrylamidomethylpropansulfonsäure und mit 25 mol Ethylenoxid polyethoxyliertem Stearylmethacrylat (Ammoniumacryloyldimethyltaurat/Steareth-25-methacrylat-Copolymer),
- einem unvernetzten Copolymer von 2-Acrylamidomethylpropansulfonsäure und einem mit 8 mol Ethylenoxid polyethoxylierten $C_{16}$/$C_{18}$-Alkylmethacrylat (Ammoniumacryloyldimethyltaurat/Steareth-8-methacrylat-Copolymer),
- einem vernetzten Copolymer von 2-Acrylamidomethylpropansulfonsäure und mit 25 mol Ethylenoxid polyethoxyliertem Behenylmethacrylat (Ammoniumacryloyldlmethyltaurat/Beheneth-25-methacrylat-Copolymer) und Mischugen davon ausgewählt ist.

**11.** Zusammensetzung nach einem der Ansprüche 6 bis 10, wobei es sich bei dem amphiphilen Polymer mit mindestens einer 2-Acrylamidomethylpropansulfonsäure-Einheit um ein Copolymer von 2-Acrylamidomethylpropansulfonsäure und einem $C_{16}$-$C_{18}$-Alkylmethacrylat mit 6 bis 25 mol Ethylenoxid handelt.

**12.** Zusammensetzung nach Anspruch 11, wobei es sich bei dem amphiphilen Polymer mit mindestens einer 2-Acrylamidomethylpropansulfonsäure-Einheit um ein Copolymer von 2-Acrylamidomethylpropansulfonsäure und einem $C_{16}$-$C_{18}$-Alkylmethacrylat mit 6 bis 10 mol Ethylenoxid, für das der Molanteil von $C_{16}$-$C_{18}$-Alkylmethacrylat-Einheiten (Einheiten (II)) im Bereich von 2 % bis 15 % und noch besser von 5 % bis 10 % beträgt, und noch spezieller ein Copolymer von 2-Acrylamidomethylpropansulfonsäure und einem Ammoniumsalz von Methacrylsäure und oxyethyleniertem Stearylalkohol mit 8 mol Ethylenoxid und einem Molanteil von Alkylmethacrylat-Einheiten von 7,35 % (Ammoniumacryloyldimethyltaurat/Steareth-8-methacrylat-Copolymer) handelt.

**13.** Zusammensetzung nach einem der vorhergehenden Ansprüche, wobei die Zusammensetzung in Form einer Öl-in-Wasser-Emulsion oder einer Wasser-in-Öl-Emulsion und spezieller in Form einer Öl-in-Wasser-Emulsion vorliegt.

**14.** Nichttherapeutisches kosmetisches Verfahren zum Pflegen und/oder Schminken eines Keratinmaterials, bei dem man auf die Oberfläche des Keratinmaterials mindestens eine Zusammensetzung gemäß einem der vorhergehenden Ansprüche aufbringt.

**15.** Nichttherapeutisches kosmetisches Verfahren zum Einschränken des Dunkelwerdens der Haut und/oder zum Verbessern der Farbe und/oder Einheitlichkeit des Teints, bei dem man auf die Oberfläche der Haut mindestens eine Zusammensetzung gemäß einem der vorhergehenden Ansprüche aufbringt.

**16.** Nichttherapeutisches kosmetisches Verfahren zum Verhindern und/oder Behandeln der Anzeichen von Alterung eines Keratinmaterials, bei dem man auf die Oberfläche des Keratinmaterials mindestens eine Zusammensetzung gemäß einem der vorhergehenden Ansprüche aufbringt.

## Revendications

**1.** Composition cosmétique ou dermatologique sous forme d'émulsion comprenant dans un support physiologiquement acceptable :

a) au moins une phase aqueuse et
b) au moins une phase huileuse et
c) au moins un composé mérocyanine de formule (1) ou l'une de ses formes géométriques isomères E/E- ou E/Z-:

(1) 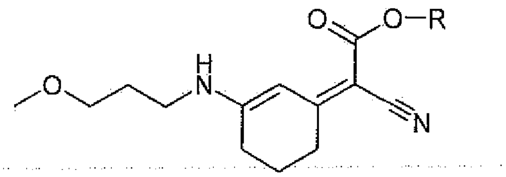

dans laquelle

R est un groupement alkyle en $C_1$-$C_{22}$, un groupement alcényle en $C_2$-$C_{22}$, un groupement alcynyle en $C_2$-$C_{22}$, un groupement cycloalkyle en $C_3$-$C_{22}$ ou un groupement cycloalcényle en $C_3$-$C_{22}$, lesdits groupements pouvant être interrompus par un ou plusieurs O et

d) au moins un système émulsionnant contenant au moins un polymère amphiphile comportant au moins un motif acide acrylamido 2-méthylpropane sulfonique.

**2.** Composition selon la revendication 1, où le ou les composés mérocyanines sont choisis parmi ceux où R est un alkyle en $C_1$-$C_{22}$, prouvant être interrompu par un ou plusieurs O.

**3.** Composition selon la revendication 1 ou 2, où le ou les composés mérocyanines sont choisis parmi les composés suivants ainsi que leurs formes géométriques isomères E/E-, E/Z- :

| | | | |
|---|---|---|---|
| 1 | 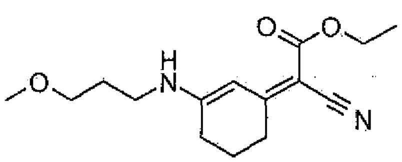<br>ethyl (2Z)-cyano{3-[(3-methoxypropyl)amino]cyclohex -2-en-1-ylidene} ethanoate | 4 | 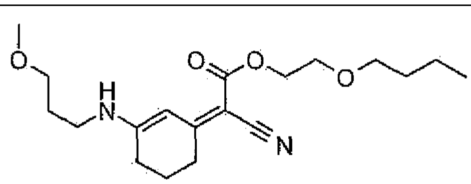<br>2-butoxyethyl (2Z)-cyano{3-[(3-methoxypropyl)amino]cyclohex -2-en-1-ylidene} ethanoate |

(suite)

| | | | |
|---|---|---|---|
| 2 | 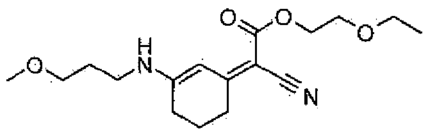<br>2-ethoxyethyl (2Z)-cyano{3-[(3-methoxypropyl)amino]cyclohex -2-en-1-ylidene} ethanoate | 5 | 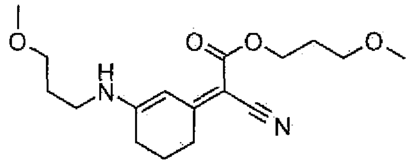<br>3-methoxypropyl (2Z)-cyano{3-[(3-methoxypropyl)amino]cyclohex -2-en-1-ylidene} ethanoate |
| 3 | 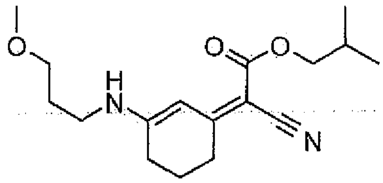<br>2-methylpropyl (2Z)-cyano{3-[(3-methoxypropyl)amino]cyclohex -2-en-1-ylidene} ethanoate | 6 | 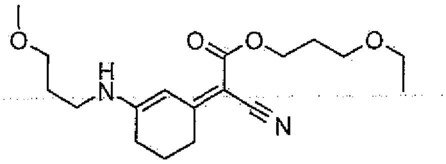<br>3-ethoxypropyl (2Z)-cyano{3-[(3-methoxypropyl)amino]cyclohex -2-en-1-ylidene} ethanoate |

**4.** Composition selon la revendication 3, où le composé mérocyanine est le 2-éthoxyéthyle (2Z)-cyano{3-[(3-méthoxypropyl)-amino]cyclohex-2-èn-1-ylidène}éthanoate (2) dans sa configuration géométrique E/Z de structure suivante :

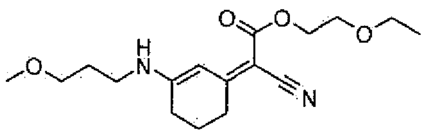

et/ou dans sa configuration géométrique E/E de structure suivante :

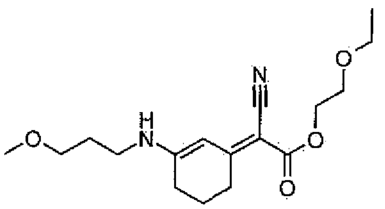

**5.** Composition selon l'une quelconque des revendications 1 à 4, où le ou les composés mérocyanines de formule (1) sont présents dans une concentration allant de 0,1% à 10% en poids, et préférentiellement de 0,2% à 5% en poids par rapport au poids total de la composition.

**6.** Composition selon l'une quelconque des revendications 1 à 4, où le polymère amphiphile comportant au moins un motif acide acrylamido 2-méthylpropane sulfonique est un polymère réticulé ou non réticulé constitué :

a) de motifs acide 2-acrylamido 2-méthylpropane sulfonique (AMPS®) de formule (II) suivante :

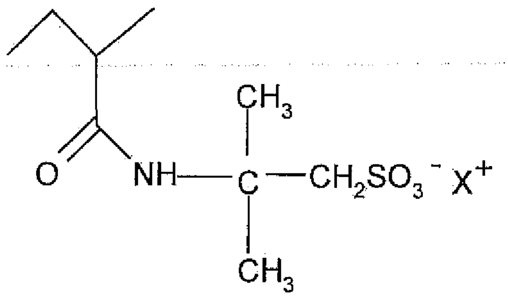

(II)

dans laquelle X est un proton, un cation de métal alcalin, un cation alcalino-terreux ou l'ion ammonium et b) de motifs de formule (III) suivante :

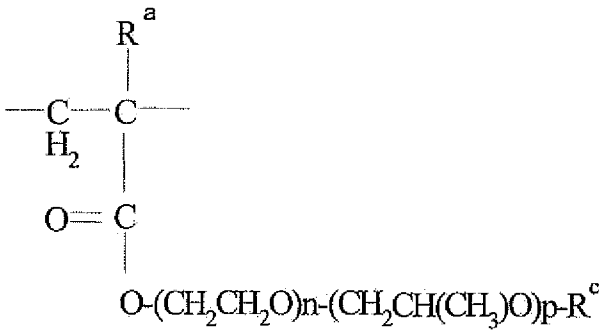

(III)

dans laquelle n et p, indépendamment l'un de l'autre désignent un nombre de moles et avarient de 0 à 30, de préférence de 1 à 25 et plus préférentiellement de 3 à 20 sous réserve que n + p soit inférieur ou égal à 30, de préférence inférieur à 25 et encore mieux inférieur à 20 ; $R^a$ désigne un atome d'hydrogène, un radical alkyle linéaire ou ramifié en $C_1$-$C_6$, de préférence méthyle et $R^c$ désigne un alkyle alinéaire ou ramifié comportant de 7 à 22 atomes de carbone, de préférence de 12 à 22 atomes de carbone.

**7.** Composition selon la revendication 6, où dans la formule (II), le cation X désigne le sodium ou l'ammonium.

**8.** Composition selon la revendication 6 ou 7, où le monomère de formule (III) est choisi parmi :

- les esters d'acide (méth)acrylique et d'alcool gras en $C_{10}$-$C_{18}$ polyoxyéthyléné à 8 moles d'oxyde d'éthylène,
- les esters d'acide (méth)acrylique et d'oxoalcool gras en $C_{11}$ polyoxyéthyléné à 8 moles d'oxyde d'éthylène,
- les esters d'acide (méth)acrylique et d'alcool gras en $C_{12}$-$C_{14}$ polyoxyéthyléné à 7 moles d'oxyde d'éthylène,
- les esters d'acide (méth)acrylique et d'alcool gras en $C_{12}$-$C_{14}$ polyoxyéthyléné à 11 moles d'oxyde d'éthylène,
- les esters d'acide (méth)acrylique et d'alcool gras en $C_{16}$-$C_{18}$ polyoxyéthyléné à 8 moles d'oxyde d'éthylène,
- les esters d'acide (méth)acrylique et d'alcool gras en $C_{16}$-$C_{18}$ polyoxyéthyléné à 15 moles d'oxyde d'éthylène,
- les esters d'acide (méth)acrylique et d'alcool gras en $C_{16}$-$C_{18}$ polyoxyéthyléné à 11 moles d'oxyde d'éthylène,
- les esters d'acide (méth)acrylique et d'alcool gras en $C_{16}$-$C_{18}$ polyoxyéthyléné à 20 moles d'oxyde d'éthylène,
- les esters d'acide (méth)acrylique et d'alcool gras en $C_{16}$-$C_{18}$ polyoxyéthyléné à 25 moles d'oxyde d'éthylène,
- les esters d'acide (méth)acrylique et d'alcool gras en $C_{18}$-$C_{22}$ polyoxyéthyléné à 25 moles d'oxyde d'éthylène et/ou d'isoalcool gras en $C_{16}$-$C_{18}$ polyoxyéthyléné à 25 moles d'oxyde d'éthylène.

**9.** Composition selon l'une quelconque des revendications 6 à 8, où le polymère amphiphile comportant au moins un motif acide acrylamido 2-méthylpropane sulfonique est choisi parmi :

- ceux non réticulés pour lesquels p = 0, n = 7 ou 25, $R^a$ désigne un méthyle et $R^c$ représente un mélange d'alkyle en $C_{12}$-$C_{14}$ ou en $C_{16}$-$C_{18}$,
- ceux réticulés pour lesquels p = 0, n = 8 ou 25, $R^a$ désigne un méthyle et $R^c$ représente un mélange d'alkyle en $C_{16}$-$C_{18}$.

**10.** Composition selon l'une quelconque des revendications 6 à 9, où le polymère amphiphile comportant au moins un motif acide acrylamido 2-méthylpropane sulfonique est choisi parmi :

- un copolymère non réticulé d'acide acrylamido 2-méthylpropane sulfonique et de méthacrylate d'alkyle en $C_{12}$-$C_{14}$ polyéthoxylé à 25 moles d'oxyde d'méthylène (Ammonium Acryloyldiméthyltaurate/Laureth-7 Methacrylate Copolymer),
- un copolymère réticulé d'acide acrylamido 2-méthylpropane sulfonique et de méthacrylate de stéaryle polyéthoxylé à 25 moles d'oxyde d'éthylène (Ammonium Acryloyldimethyltaurate/Steareth-25 Methacrylate Crosspolymer),
- un copolymère non réticulé d'acide acrylamido 2-méthylpropane sulfonique et de méthacrylate d'alkyle en $C_{16}$-$C_{18}$ polyéthoxylé à 8 moles d'oxyde d'éthylène (Ammonium Acryloyldiméthyltaurate/Steareth-8 Methacrylate Copolymer)
- un copolymère réticulé d'acide acrylamido 2-méthylpropane sulfonique et de méthacrylate de béhényle polyéthoxylé à 25 moles d'oxyde d'éthylène (Ammonium Acryloyldimethyltaurate/Beheneth-25 Methacrylate Crosspolymer) et leurs mélanges.

**11.** Composition selon l'une quelconque des revendications 6 à 10, où le polymère amphiphile comportant au moins un motif acide acrylamido 2-méthylpropane sulfonique est un copolymère d'acide acrylamido 2-méthylpropane sulfonique et de méthacrylate d'alkyle en $C_{16}$-$C_{18}$ comportant de 6 à 25 moles d'oxyde d'éthylène.

**12.** Composition selon la revendication 11, où le polymère amphiphile comportant au moins un motif acide acrylamido 2-méthylpropane sulfonique est un copolymère d'acide acrylamido 2-méthylpropane sulfonique et de méthacrylate d'alkyle en $C_{16}$-$C_{18}$ comportant de 6 à 10 moles d'oxyde d'éthylène, dont la proportion molaire d'unités méthacrylate d'alkyle en $C_{16}$-$C_{18}$ (motifs (II)) va de 2% à 15%, et encore mieux de 5% à 10%, et encore plus particulièrement un copolymère d'acide acrylamido 2-méthylpropane sulfonique et d'un sel d'ammonium d'acide méthacrylique et d'alcool stéarylique oxyéthyléné comportant 8 moles d'oxyde d'éthylène et comportant une proportion molaire d'unités méthacrylate d'alkyle de 7,35 %, (Ammonium Acryloyldimethyltaurate/Steareth-8 Methacrylate Copolymer).

**13.** Composition selon l'une quelconque des revendications précédentes, où la composition se présente sous la forme d'une émulsion huile-dans-eau ou d'une émulsion eau-dans-huile et plus particulièrement sous la forme d'une émulsion huile-dans-eau.

**14.** Procédé cosmétique non-thérapeutique de soin et/ou de maquillage d'une matière kératinique comprenant l'application sur la surface de ladite matière kératinique d'au moins une composition telle que définie dans l'une quelconque des revendications précédentes.

**15.** Procédé cosmétique non-thérapeutique pour limiter l'assombrissement de la peau et/ou améliorer la couleur et/ou l'homogénéité du teint comprenant l'application sur la surface de la peau d'au moins une composition telle que définie dans l'une quelconque des revendications précédentes

**16.** Procédé cosmétique non-thérapeutique pour prévenir et/ou traiter les signes du vieillissement d'une matière kératinique comprenant l'application sur la surface de la matière kératinique d'au moins une composition telle que définie dans l'une quelconque des revendications précédentes.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4195999 A
- WO 2004006878 A
- WO 2008090066 A
- WO 2011113718 A
- WO 2009027258 A
- WO 2013010590 A
- WO 2013011094 A
- WO 2013011480 A
- EP 1728501 A1
- DE 102005059738 A1
- WO 2007071582 A
- US 4749643 A
- WO 0031154 A
- EP 0750899 A
- US 5089578 A
- EP 1069142 A
- WO 0244224 A
- WO 0244225 A
- WO 0244227 A
- WO 0244229 A
- WO 0244230 A
- WO 0244231 A
- WO 0244267 A
- WO 0244268 A
- WO 0244269 A
- WO 0244270 A
- WO 0244271 A
- WO 0243677 A
- WO 0243686 A
- WO 0243687 A
- WO 0243688 A
- WO 0243689 A
- WO 9206778 A
- WO 2007068371 A
- WO 2008155059 A
- US 5624663 A
- EP 669323 A
- US 2463264 A
- US 5237071 A
- US 5166355 A
- GB 2303549 A
- DE 19726184
- EP 893119 A
- EP 0832642 A
- EP 1027883 A
- EP 1300137 A
- DE 10162844
- WO 9304665 A
- DE 19855649
- EP 0967200 A
- DE 19746654
- DE 19755649
- EP 1008586 A
- EP 1133980 A
- EP 133981 A
- WO 2007071584 A
- WO 2009063392 A
- US 6225467 B
- WO 2004085412 A
- WO 06035000 A
- WO 06034982 A
- WO 06034991 A
- WO 06035007 A
- WO 2006034992 A
- WO 2006034985 A
- EP 0841341 A
- EP 0518773 A
- FR 2315991
- FR 2416008
- US 4077441 A
- US 4850517 A


### Non-patent literature cited in the description


- *IP COM JOURNAL,* 23 February 2009
- *IP COM JOURNAL*
- *IP COM JOURNAL,* 12 November 2009
- *IP COM Journal,* 04 March 2004
- **W.C. GRIFFIN.** Classification of Surface Active Agents by HLB. *Journal of the Society of Cosmetic Chemists,* 1949, vol. 1, 311
- Calculation of HLB of Non Ionic Surfactants. *Journal of the Society of Cosmetic Chemists,* 1954, vol. 5, 249
- *IP.com Journal,* 2009, vol. 9 (5A), 29-30
- Self-assembling amphiphilic polyelectrolytes and their nanostructures. *Journal of Polymer Science,* 2000, vol. 18 (40), 323-336

- Micelle formation of random copolymers of sodium 2-(acrylamido)-2-methylpropanesulfonate and a nonionic surfactant macromonomer in water as studied by fluorescence and dynamic light scattering. *Macromolecules,* 2000, vol. 33 (10), 3694-3704
- Solution properties of micelle networks formed by nonionic moieties covalently bound to a polyelectrolyte: salt effects on rheological behavior. *Langmuir,* 2000, vol. 16 (12), 5324-5332
- Stimuli responsive amphiphilic copolymers of sodium 2-(acrylamido)-2-methylpropanesulfonate and associative macromonomers. *Polym. Preprint, Div. Polym. Chem.,* 1999, vol. 40 (2), 220-221
- *CHEMICAL ABSTRACTS,* 919803-06-8
- **BANGHAM ; STANDISH ; WATKINS.** *J. Mol. Biol.,* 1965, vol. 13, 238
- **B.L. DIFFEY.** *J. Soc. Cosmet. Chem.,* 1989, vol. 40, 127-133